(19) Europäisches Patentamt
European Patent Office
Office européen des brevets

(11) EP 1 818 322 A1

(12) EUROPEAN PATENT APPLICATION
published in accordance with Art. 158(3) EPC

(43) Date of publication:
**15.08.2007 Bulletin 2007/33**

(21) Application number: **05809745.2**

(22) Date of filing: **25.11.2005**

(51) Int Cl.:
***C07C 15/38*** *(2006.01)*

(86) International application number:
**PCT/JP2005/021647**

(87) International publication number:
**WO 2006/057325 (01.06.2006 Gazette 2006/22)**

(84) Designated Contracting States:
**DE**

(30) Priority: **25.11.2004 JP 2004340944**
**28.09.2005 JP 2005282590**

(71) Applicants:
- **ROHM CO., LTD.**
  **Kyoto-shi, Kyoto 615-8585 (JP)**
- **Pioneer Corporation**
  **Tokyo 153-8654 (JP)**
- **HITACHI, LTD.**
  **Chiyoda-ku**
  **Tokyo 100-8280 (JP)**
- **Mitsubishi Chemical Corporation**
  **Minato-ku**
  **Tokyo 108-0014 (JP)**

(72) Inventors:
- **OYAMADA, Takahito**
  **Tsurugashima-shi, Saitama, 3502201 (JP)**
- **UCHIUZOU, Hiroyuki,**
  **Kyushu University**
  **Fukuoka-shi, Fukuoka 8128581 (JP)**
- **ADACHI, Chihaya,**
  **Kyushu University**
  **Fukuoka-shi, Fukuoka 8128581 (JP)**
- **AKIYAMA, Seiji,**
  **Mitsubishi Chemical Group**
  **Aoba-ku, Yokohama-shi, Kanagawa 2278502 (JP)**
- **TAKAHASHI, Takayoshi,**
  **Graduate school of Engineering**
  **Kyoto-shi, Kyoto 6158510 (JP)**

(74) Representative: **Grünecker, Kinkeldey,**
**Stockmair & Schwanhäusser**
**Anwaltssozietät**
**Maximilianstrasse 58**
**80538 München (DE)**

# (54) PYRENE COMPOUND AND, UTILIZING THE SAME, LIGHT EMITTING TRANSISTOR DEVICE AND ELECTROLUMINESCENCE DEVICE

(57) It is an object to provide an organic fluorescent substance which can be used in a light emitting transistor element and an organic EL element.

The invention provides a light emitting transistor element or an organic electroluminescence element wherein a specific asymmetric pyrene based compound is used in a light emitting layer in the light emitting transistor element, or in a light emitting layer, a hole transporting layer or an electron transporting layer in the organic EL element.

EP 1 818 322 A1

## Description

TECHNICAL FIELD

The present invention relates to an asymmetric pyrene based compound which can be used in both of a light emitting transistor element and an organic electroluminescence element, and a light emitting transistor element and an organic electroluminescence element using the same.

BACKGROUND ART

Organic electroluminescence elements (hereinafter abbreviated to "organic EL elements"), which are typical examples of organic semiconductor devices, are light emitting elements using a light emitting phenomenon based on recombination of electrons and holes in a layer made of an organic fluorescent substance. Specifically, Patent Documents 1 and 2 and others describe organic EL elements each consisting of a light emitting layer made of the abovementioned organic compound, an electron injecting electrode for injecting electrons into this light emitting layer, and a hole injecting electrode for injecting holes into the light emitting layer.
Examples of the organic fluorescent substance used in this light emitting layer include perynone derivatives, distyrylbenzene derivatives (Patent Document 1), and 1,3,6,8-tetraphenylpyrene (Patent Document 2).
Patent Document 1: JP-A-5-315078
Patent Document 2: JP-A-2001-118682

DISCLOSURE OF THE INVENTION

PROBLEMS TO BE SOLVED BY THE INVENTION

On the other hand, besides such organic EL elements, light emitting transistor elements are known as examples using a light emitting phenomenon based on recombination of electrons and holes in a layer made of an organic fluorescent substance.
If an organic fluorescent substance can be used in both of an organic EL element and a light emitting transistor element, the efficiency of the production of these elements can be increased. However, no organic fluorescent substance is known which can be used in both of these elements.
Thus, it is an object of the present invention to provide an organic fluorescent substance which can be used in both of a light emitting transistor element and an organic EL element.

MEANS FOR SOLVING THE PROBLEMS

The present invention solves the above-mentioned problems by use of an asymmetric pyrene based compound represented by the following structural formula (1):

[Chemical formula 4]

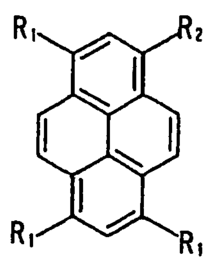

(1)

(wherein $R_1$ represents a group selected from a heteroaryl group which may have a substituent, an aryl group which may have a substituent, an alkyl group which may have a substituent and has a main chain having 1 to 20 carbon

atoms, a cycloalkyl group which may have a substituent, an alkenyl group which may have a substituent, an alkynyl group which may have a substituent, a cyano group, a carbonyl group which may have a substituent, an alkoxy group which may have a substituent, an aryloxy group which may have a substituent, a silyl group which may have a substituent, an aryl boryl group which may have a substituent, an ester group which may have a substituent, and halogen atoms.

$R_2$ represents a group selected from a hydrogen atom, a heteroaryl group which may have a substituent, an aryl group which may have a substituent, an alkyl group which may have a substituent and has a main chain having 1 to 20 carbon atoms, a cycloalkyl group which may have a substituent, an alkenyl group which may have a substituent, an alkynyl group which may have a substituent, a cyano group, a carbonyl group which may have a substituent, an alkoxy group which may have a substituent, an aryloxy group which may have a substituent, a silyl group which may have a substituent, an aryl boryl group which may have a substituent, an ester group which may have a substituent, and halogen atoms, and is different from $R_1$.)

EFFECT OF THE INVENTION

According to the present invention, since an asymmetric compound is used as a pyrene based compound, the pyrene based compound can be used as an organic fluorescent substance used in an organic EL element and a light emitting transistor element. This is presumably because the asymmetric pyrene based compound has an appropriate crystallinity (amorphousness) and thus the compound has an appropriate luminous brightness for an organic EL element and further has an appropriate carrier mobility for a light emitting transistor element.

BRIEF DESCRIPTION OF THE DRAWINGS

Fig. 1(a) is chemical formulae showing examples of the asymmetric pyrene based compound.
Fig. 1(b) is chemical formulae showing examples of the asymmetric pyrene based compound.
Fig. 2(a) is chemical formulae showing examples of the asymmetric pyrene based compound.
Fig. 2(b) is chemical formulae showing examples of the asymmetric pyrene based compound.
Fig. 2(c) is chemical formulae showing examples of the asymmetric pyrene based compound.
Fig. 3(a) is chemical formulae showing examples of the asymmetric pyrene based compound.
Fig. 3(b) is chemical formulae showing examples of the asymmetric pyrene based compound.
Fig. 3(c) is chemical formulae showing examples of the asymmetric pyrene based compound.
Fig. 4(a) is chemical formulae showing examples of the asymmetric pyrene based compound.
Fig. 4(b) is chemical formulae showing examples of the asymmetric pyrene based compound.
Fig. 5 is a sectional view illustrating an example of a light emitting transistor element according to the present invention.
Fig. 6 is a plan view illustrating a structure of a source electrode and a drain electrode.
Figs. 7 (a), (b) and (c) are schematic views illustrating the mechanism of light emission of a light emitting transistor element.
Fig. 8 is an electric circuit diagram illustrating an example of a display device wherein a light emitting transistor element according to the present invention is used.
Fig. 9 is a sectional view illustrating an example of an organic EL element according to the present invention.
Fig. 10 is an electric circuit diagram illustrating an example of a display element wherein an organic EL element according to the present invention is used.

| | |
|---|---|
| 1 | Light emitting layer |
| 2 | Source electrode |
| 2a | Comb tooth shaped region |
| 3 | Drain electrode |
| 3a | Comb tooth shaped region |
| 4 | Gate electrode |
| 5 | Insulating film |
| 10 | Light emitting transistor element |
| 11 | Hole channel |
| 12 | Pinch-off point |
| 20 | Substrate |

21 Display device
22 Scanning line driving device
23 Data line driving device
24 Controller

30 Organic EL element
31 Substrate
32 Hole injecting electrode layer
33 Hole transporting layer
34 Light emitting layer
35 Electron transporting layer
36 Electron injecting electrode layer
37 Direct current power source

40 Substrate
41 Display device
42 Scanning line driving circuit
43 Data line driving circuit

| | |
|---|---|
| S | Source electrode |
| D | Drain electrode |
| G | Gate electrode |
| C | Capacitor |
| Ts | Selecting transistor |
| P11 & P12 | Pixels |
| LS1, LS2, LS1' & LS2' | Scanning lines |
| LD1, LD2, LD1' & LD2' | Data lines |

BEST MODE FOR EMBODYING THE INVENTION

The present invention will be described in detail hereinafter.
The present invention relates to an asymmetric pyrene based compound. This asymmetric pyrene based compound can be used in a light emitting transistor element or an organic electroluminescence element (organic EL element).
The asymmetric pyrene based compound is a compound represented by the following chemical formula (1):
[Chemical formula 5]

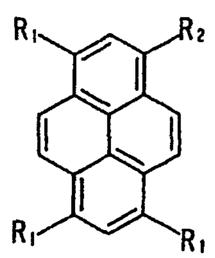

(1)

In the formula, $R_1$ represents a group selected from a heteroaryl group which may have a substituent, an aryl group which may have a substituent, an alkyl group which may have a substituent and has a main chain having 1 to 20 carbon atoms, a cycloalkyl group which may have a substituent, an alkenyl group which may have a substituent, an

alkynyl group which may have a substituent, a cyano group, a carbonyl group which may have a substituent, an alkoxy group which may have a substituent, an aryloxy group which may have a substituent, a silyl group which may have a substituent, an aryl boryl group which may have a substituent, an ester group which may have a substituent, and halogen atoms.

$R_2$ represents a group selected from a hydrogen atom, a heteroaryl group which may have a substituent, an aryl group which may have a substituent, an alkyl group which may have a substituent and has a main chain having 1 to 20 carbon atoms, a cycloalkyl group which may have a substituent, an alkenyl group which may have a substituent, an alkynyl group which may have a substituent, a cyano group, a carbonyl group which may have a substituent, an alkoxy group which may have a substituent, an aryloxy group which may have a substituent, a silyl group which may have a substituent, an aryl boryl group which may have a substituent, an ester group which may have a substituent, and halogen atoms, and is different from $R_1$.

Specific examples of $R_1$ described above include a heteroaryl group which may have a predetermined substituent (which may be a polycyclic aromatic group), an alkylphenyl group substituted with a predetermined alkyl group, a phenyl group substituted with a halogen atom, a naphthyl group (preferably, 2-naphthyl group), an anthryl group (preferably, 2-anthryl group), a phenanthryl group, an aryl group which may have a predetermined substituent (which may be a polycyclic aromatic group), a linear or branched alkyl group which has a main chain having 1 to 20 carbon atoms and may have a substituent, a cycloalkyl group which may have a substituent, an alkenyl group which may have a predetermined substituent, an alkynyl group which may have a predetermined substituent, a cyano group, a carbonyl group which may have a substituent, an alkoxy group which may have a predetermined substituent, an aryloxy group which may have a predetermined substituent, a silyl group which may have a substituent, such as a trimethylsilyl group, an aryl boryl group which may have a substituent, an ester group which may have a substituent, and a group having a halogen atom.

In the above-mentioned heteroaryl group which may have a predetermined substituent, examples of the substituent used therein include benzofuryl, pyrrolyl, benzoxazolyl, pyrazinyl, thienyl, alkyl-substituted thienyl, bithienyl, phenylthienyl, benzothienyl, pyridyl, bipyridyl, phenylpyridyl, quinolyl, and benzothiazolyl groups.

In the above-mentioned alkylphenyl group substituted with a predetermined alkyl group, examples of the alkyl group used therein include phenyl, methyl, and ethyl groups. Specific examples of the alkylphenyl group substituted with an alkyl group include tolyl, 3-alkylphenyl, 4-alkylphenyl, 3-trifluoromethylphenyl, 4-trifluoromethylphenyl, and 3,5-bis(trifluoromethyl)phenyl groups.

In the above-mentioned phenyl group substituted with a halogen atom, examples of the halogen atom used therein include fluorine, bromine and chlorine atoms. A fluorine atom is preferred. Specific examples of the phenyl group substituted with a halogen atom include 3-fluorophenyl, 4-fluorophenyl, 3,4-difluorophenyl, 3,5-difluorophenyl and 3,4,5-trifluorophenyl groups.

In the above-mentioned aryl group which may have a predetermined substituent, examples of the substituent used therein include biphenyl, terphenyl, phenyl-etheno-phenyl, and pyridino-phenyl groups.

Specific examples of the above-mentioned linear or branched alkyl group which has a main chain having 1 to 20 carbon atoms and may have a substituent include methyl, ethyl, n-propyl, 2-propyl, n-butyl, isobutyl, tert-butyl, hexyl, octyl, dodecyl, and octadecyl groups.

In the above-mentioned alkenyl group, which may have a predetermined substituent, examples of the substituent used therein include vinyl, phenyl-substituted vinyl, ethyl-substituted vinyl, biphenyl-substituted vinyl, allyl, and 1-butenyl groups.

In the above-mentioned alkynyl group, which may have a predetermined substituent, examples of the substituent used therein include ethynyl, phenyl-substituted ethynyl, trimethylsilyl-substituted ethynyl, and propargyl groups.

In the above-mentioned alkoxy group which may have a predetermined substituent, examples of the substituent used therein include methoxy, ethoxy, and butoxy groups.

In the above-mentioned aryloxy group which may have a predetermined substituent, examples of the substituent used therein include phenyloxy, 1-naphthyloxy and 2-naphthyloxy groups.

In the above-mentioned group having a halogen atom, examples of the halogen atom used therein include fluorine, bromine, and chlorine atoms. A group consisting only of a halogen atom is preferable, and a fluorine atom is particularly preferable.

Of the above-mentioned groups, $R_1$ is preferably a group which is selected from phenyl, naphthyl, benzofuryl, phenylpyridyl, thienyl, benzothienyl, pyridyl, methyl, vinyl and ethynyl groups and which may have a substituent.

Specifically, $R_1$ is particularly preferably a group selected from 3-alkylphenyl, 4-alkylphenyl, 3-fluorophenyl, 4-fluorophenyl, 3-trifluoromethylphenyl, 4-trifluoromethylphenyl, 3,4-difluorophenyl, 3,5-difluorophenyl, 3,5-bis(trifluoromethyl)phenyl, 3,4,5-trifluorophenyl, tolyl, fluorine-substituted phenyl, 2-naphthyl, benzofuryl, phenylpyridyl, thienyl, benzothienyl, pyridyl, bipyridyl, phenyl, biphenyl, methyl, phenyl-substituted vinyl and phenyl-substituted ethynyl groups.

Specific examples of $R_2$ described above include a hydrogen atom, and the groups exemplified as $R_1$ described above. In the invention, $R_1$ is different from $R_2$.

$R_2$ is preferably a group which is selected from a hydrogen atom, and phenyl, naphthyl, benzofuryl, phenylpyridyl, thienyl, benzothienyl, pyridyl, methyl, vinyl and ethynyl groups, and which may have a substituent.

Specifically, $R_2$ is particularly preferably a group selected from a hydrogen atom, and methyl, hexyl, phenyl, biphenyl, pyridyl, bipyridyl, naphthyl, biphenyl, phenylpyridyl, octyl, dodecyl, octadecyl, phenyl-substituted vinyl and phenyl-substituted ethynyl group.

The molecular weight of the asymmetric pyrene based compound of the invention is preferably 500 or more, more preferably 800 or more, and is preferably 5000 or less, more preferably 3000 or less.

Specific examples of the chemical formula (1) include compounds as described hereinafter. Examples of the compound wherein $R_2$ is a hydrogen atom include a pyrene based compound wherein $R_1$ is a thiophene ring (thienyl group) ((3-1) in Fig. 1(a)), a pyrene based compound wherein $R_1$ is a pyridine ring (pyridyl group) ((3-2) in Fig. 1(a)), a pyrene based compound wherein $R_1$ is a phenyl group ((3-3) in Fig. 1(a)), a pyrene based compound wherein $R_1$ is a naphthyl group ((3-4) in Fig. 1(a)), a pyrene based compound wherein $R_1$ is a biphenyl group ((3-5) in Fig. 1(a)), a pyrene based compound wherein $R_1$ is a methyl group ((3-6) in Fig. 1(a)), a pyrene based compound wherein $R_1$ is a phenyl-substituted vinyl group ((3-7) in Fig. 1(a)), a pyrene based compound wherein $R_1$ is a phenyl-substituted ethynyl group ((3-8) in Fig. 1(a)), a pyrene based compound wherein $R_1$ is a biphenyl group ((3-9) in Fig. 1(b)), a pyrene based compound wherein $R_1$ is a benzothiophene ring (benzothienyl group) ((3-10) in Fig. 1(b)), a pyrene based compound wherein $R_1$ is a tolyl group ((3-11) in Fig. 1(b)), and a pyrene based compound wherein $R_1$ is a fluorine-substituted phenyl group ((3-12) in Fig. 1(b)).

Examples of the compound wherein $R_2$ is other than a hydrogen atom include a pyrene based compound wherein $R_1$ is a pyridine ring (pyridyl group) and $R_2$ is a methyl group ((4-1) in Fig. 2(a)), a pyrene based compound wherein $R_1$ is a bipyridyl group and $R_2$ is an octadecyl group ((4-2) in Fig. 2(a)), a pyrene based compound wherein $R_1$ is a phenyl group and $R_2$ is a methyl group ((4-3) in Fig. 2(a)), a pyrene based compound wherein $R_1$ is a phenyl group and $R_2$ is an octyl group ((4-4) in Fig. 2(a)), a pyrene based compound wherein $R_1$ is a phenyl group and $R_2$ is a dodecyl group ((4-5) in Fig. 2(a)), a pyrene based compound wherein $R_1$ is a biphenyl group and $R_2$ is an octyl group ((4-6) in Fig. 2(a)), a pyrene based compound wherein $R_1$ is a biphenyl group and $R_2$ is a dodecyl group ((4-7) in Fig. 2(a)), a pyrene based compound wherein $R_1$ is a naphthyl group and $R_2$ is a dodecyl group ((4-8) in Fig. 2(b)), a pyrene based compound wherein $R_1$ is a phenyl group and $R_2$ is a phenyl-substituted vinyl group ((4-9) in Fig. 2(b)), a pyrene based compound wherein $R_1$ is a thiophene ring (thienyl group) and $R_2$ is a pyridine ring (pyridyl group) ((4-10) in Fig. 2(b)), a pyrene based compound wherein $R_1$ is a phenyl group and $R_2$ is a pyridine (pyridyl group) ((4-11) in Fig. 2(b)), a pyrene based compound wherein $R_1$ is a biphenyl group and $R_2$ is a phenylpyridine ring (phenylpyridyl group) ((4-12) in Fig. 2 (b)), pyrene based compounds wherein $R_1$ is a biphenyl group and $R_2$ is a pyridine ring (pyridyl group) ((4-13) to (4-14) in Fig. 2(b)), a pyrene based compound wherein $R_1$ is a pyridine ring (pyridyl group) and $R_2$ is a phenyl group ((4-15) in Fig. 2(c)), a pyrene based compound wherein $R_1$ is a bipyridyl group and $R_2$ is a biphenyl group ((4-16) in Fig. 2(c)), a pyrene based compound wherein $R_1$ is a biphenyl group and $R_2$ is a bipyridyl group ((4-17) in Fig. 2(c)), and a pyrene based compound wherein $R_1$ is a phenyl group and $R_2$ is a pyridine ring (pyridyl group) ((4-18) in Fig. 2(c)).

A different example of the above-mentioned asymmetric pyrene based compound is a compound represented by the following formula (2), wherein $R_2$ has, in a part thereof, a pyrene ring which may have a substituent:

[Chemical formula 6]

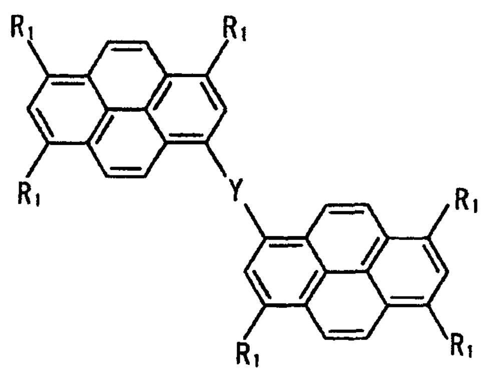

(2)

In the formula (2), $R_1$ is the same as in the formula (1), and Y represents a bivalent linking group. Specifically, Y may be a bivalent one of the groups exemplified above as $R_2$ (except groups that cannot turn to bivalent groups, such as a hydrogen atom and halogen atoms). Y is particularly preferably a group selected from bivalent groups derived from aromatic heterocyclic rings (heteroaryls), aromatic hydrocarbon rings (aryls), alkanes, alkenes, and an alkynes. The

bivalent groups may have a substituent. In the invention, the aromatic heterocyclic ring and the aromatic hydrocarbon ring mean rings which are polycyclic aromatic.

Specific examples of Y described above include bivalent groups derived from benzene rings (such as phenylene, biphenylene, and terphenylene groups), bivalent groups derived from naphthalene rings, bivalent groups derived from anthracene rings, bivalent groups derived from fluorene rings, bivalent groups derived from pyrazine rings, bivalent groups derived from pyridine rings, bivalent groups derived from carbazole rings, bivalent groups derived from thiophene rings, bivalent groups derived from thiazole rings, and bivalent groups derived from acetylene. Any of them may have a substituent. Any of these groups may a group wherein two or more rings are coupled together (such as a biphenylene group).

Examples of the asymmetric pyrene based compound represented by formula (2) include a pyrene based compound wherein $R_1$ is a phenyl group and Y is a bivalent phenyl group ((5-1) in Fig. 3(a)), a pyrene based compound wherein $R_1$ is a phenyl group and Y is a bivalent pyrazine group ((5-2) in Fig. 3(a)), a pyrene based compound wherein $R_1$ is a phenyl group and Y is a bivalent pyridine ring (pyridyl group) ((5-3) in Fig. 3(a)), a pyrene based compound wherein $R_1$ is a methyl group and Y is a bivalent phenyl group ((5-4) in Fig. 3(a)), a pyrene based compound wherein $R_1$ is a methyl group and Y is a bivalent phenyl group substituted with two dodecyl ether groups ((5-5) in Fig. 3(a)), a pyrene based compound wherein $R_1$ is a tolyl group and Y is a bivalent acetylene group ((5-6) in Fig. 3(b)), a pyrene based compound wherein $R_1$ is a methyl group and Y is a bivalent bipyridyl group ((5-7) in Fig. 3(b)), a pyrene based compound wherein $R_1$ is a methyl group and Y is a bivalent biphenyl group ((5-8) in Fig. 3(b)), a pyrene based compound wherein $R_1$ is a pyridine ring (pyridyl group) and Y is a bivalent substituted biphenyl group ((5-9) in Fig. 3(c)), a pyrene based compound wherein $R_1$ is a phenyl group and Y is a bivalent biphenyl group ((5-10) in Fig. 3(c)), and a pyrene based compound wherein $R_1$ is a phenyl group and Y is a bivalent bipyridyl group ((5-11) in Fig. 3(c)).

Furthermore, other examples of the asymmetric pyrene used in the present invention include (6-1) to (6-11) shown in Figs. 4(a) and (b).

The asymmetric pyrene based compound can be produced by a process of the following reaction formula <1>:

[Chemical formula 7]

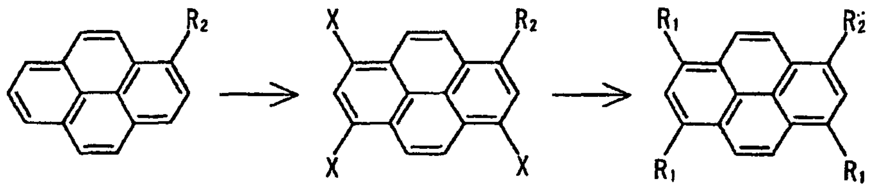

⟨1⟩

(1-1) (1-2) (1)

wherein $R_1$ and $R_2$ have the same meanings as in the case of the above-mentioned (1), and X represents a halogen atom selected from chlorine, bromine and iodine atoms.

Specifically, the asymmetric pyrene based compound (1) can be produced by reacting 1-substituted pyrene (1-1), which is substituted with $R_2$ at position 1, with halides, thereby synthesizing 1-substituted-3,6,8-trihalopyrene (1-2), which is substituted with $R_2$ at position 1 and substituted with the halogens at positions 3, 6 and 8, and by reacting the resultant with an organometallic compound selected from a borate compound, a tin compound, an organic zinc compound, and an organic magnesium compound.

The asymmetric pyrene based compound is used as a component of: a light emitting layer in a light emitting transistor element; a light emitting layer, a hole transporting layer or an electron transporting layer in an organic EL element; or the like. More specifically, the asymmetric pyrene based compound is used as a main component of a light emitting layer in a light emitting transistor element, or a light emitting layer in an organic EL element, and is used as a main component or a dopant material of a hole transporting layer or an electron transporting layer in an organic EL element. The light emitting layer is a layer which emits light by recombination of holes and electrons. The hole transporting layer and the electron transporting layer will be described later.

The above-mentioned main component means a component which takes a leading part for exhibiting luminous brightness, luminous efficiency, carrier mobility, peculiar light color, and other effects. The above-mentioned dopant is a kind of secondary component added to the main component, and means a compound added to improve the performance of the main component. When the asymmetric pyrene based compound is used as the main component, secondary

components such as a different organic fluorescent substance and a dopant may be optionally used together in order to improve the above-mentioned effects.

Such a different organic fluorescent substance is not particularly limited, and examples thereof include condensed ring derivatives such as anthracene, phenanthrene, pyrene, perylene and chrysene, metal complexes of quinolinol derivatives, such as tris(8-quinolinolato)aluminum, benzoxazole derivatives, stilbene derivatives, benzthiazole derivatives, thiadiazole derivatives, thiophene derivatives, tetraphenylbutadiene derivatives, cyclopentadiene derivatives, oxadiazole derivatives, bisstyryl derivatives such as bisstyrylanthracene and distyrylbenzene derivatives, metal complexes wherein a quinolinol derivative is combined with a different ligand, oxadiazole derivative metal complexes, benzazole derivative metal complexes, coumarin derivatives, pyrrolopyridine derivatives, perynone derivatives, and thiadiazolopyridine derivatives. Other examples of the organic fluorescent substance of a polymeric type include polyphenylene vinylene derivatives, polyparaphenylene derivatives, and polythiophene derivatives.

The above-mentioned dopant material is not particularly limited, and examples thereof include condensed ring derivatives such as phenanthrene, anthracene, pyrene, tetracene, pentacene, perylene, naphthopyrene, dibenzopyrene and rubrene, benzoxazole derivatives, benzthiazole derivatives, benzimidazole derivatives, benztriazole derivatives, oxazole derivatives, oxadiazole derivatives, thiazole derivatives, imidazole derivatives, thiadiazole derivatives, triazole derivatives, pyrazoline derivatives, stilbene derivatives, thiophene derivatives, tetraphenylbutadiene derivatives, cyclopentadiene derivatives, bisstyryl derivatives such as bisstyrylanthracene derivatives and distyrylbenzene derivatives, diazaindacene derivatives, furan derivatives, benzofuran derivatives, isobenzofuran derivatives such as phenylisobenzofuran, dimesitylisobenzofuran, di(2-methylphenyl)-isobenzofuran, di(2-trifluoromethylphenyl)isobenzofuran and phenylisobenzofuran, dibenzofuran derivatives, coumarin derivatives such as 7-dialkylaminocoumarin derivatives, 7-piperidinocoumarin derivatives, 7-hydroxycoumarin derivatives, 7-methoxycoumarin derivatives, 7-acetoxycoumarin derivatives, 3-benzthiazolylcoumarin derivatives, 3-benzimidazolylcoumarin derivatives and 3-benzoxazolylcoumarin derivatives, dicyanomethylenepyran derivatives, dicyanomethylene-thiopyran derivatives, polymethine derivatives, cyanine derivatives, oxobenzanthracene derivatives, xanthene derivatives, rhodamine derivatives, fluorescein derivatives, pyrylium derivatives, carbostyryl derivatives, acridine derivatives, bis(styryl)benzene derivatives, oxazine derivatives, phenylene oxide derivatives, quinacridone derivatives, quinazoline derivatives, pyrrolopyridine derivatives, furopyridine derivatives, 1,2,5-thiadiazolopyrene derivatives, perynone derivatives, pyrrolopyrrole derivatives, squalirium derivatives, violanthrone derivatives, phenazine derivatives, acridone derivatives, and diazaflavin derivatives.

Description is now made of a light emitting transistor element using the above-mentioned pyrene based compound.

The light emitting transistor element may be an element having a basic structure of a field effect transistor (FET) as illustrated in Fig. 5.

This light emitting transistor element 10 comprises a light emitting layer 1 which is capable of transporting holes and electrons as carriers, which emits light by recombination of the holes and the electrons, and which contains the above-mentioned pyrene based compound as a main component; a hole injecting electrode for injecting holes into this light emitting layer 1, i.e., what is called a source electrode 2; an electron injecting electrode for injecting electrons into the light emitting layer, i.e., what is called a drain electrode 3; and a gate electrode 4 which is provided opposite to the source electrode 2 and the drain electrode 3 and is made of an $N^+$ silicon substrate to control the distribution of the carriers in the light emitting layer 1. The gate electrode 4 may be made of an electroconductive layer comprising an impurity diffusion layer formed on the surface of the silicon substrate.

Specifically, as shown in Fig. 5, an insulating film 5 made of silicon oxide or the like is formed on the gate electrode 4, and the source electrode 2 and the drain electrode 3 are formed thereon at an interval. The light emitting layer 1 is formed to cover the source electrode 2 and the drain electrode 3 and to be disposed between the two electrodes.

In order for the above-mentioned element to exhibit the function of the light emitting transistor, it is preferred that the difference between the HOMO energy level and the LUMO energy level of the organic fluorescent substance which constitutes the light emitting layer 1, in particular, the pyrene based compound as the main component thereof, the carrier mobility thereof, or the luminous efficiency thereof satisfies a predetermined range. When the pyrene based compound having the above-mentioned individual characteristics is used, it is possible to improve the individual functions by adding the above-mentioned secondary constituting component, such as the dopant, thereto.

First, the difference between the HOMO energy level and the LUMO energy level is preferably as small as possible so that the electrons can move more easily, and thus the light emission and the semi-conductivity (that is, the conductivity of electrons or holes in one direction) can be generated more easily. Specifically, the difference is preferably 5 eV or less, more preferably 3 eV or less, even more preferably 2.7 eV or less. Because the smaller this difference, the better the results, the lower limit of this difference is 0 eV.

The carrier mobility is preferably as high as possible for improved semi-conductivity. Specifically, the carrier mobility is preferably $1.0 \times 10^{-5}$ $cm^2/V \cdot s$ or more, more preferably $4.0 \times 10^{-5}$ $cm^2/V \cdot s$ or more, even more preferably $1.0 \times 10^{-4}$ $cm^2/V \cdot s$ or more. The upper limit of the carrier mobility is not particularly limited, and it is sufficient if the upper limit is about 1 $cm^2/V \cdot s$.

The above-mentioned luminous efficiency means the ratio of light generated by the injection of photons or electrons. The ratio of emitted optical energy to injected optical energy is defined as the PL luminous efficiency (or PL quantum efficiency), and the ratio of the number of emitted photons to the number of injected electrons is defined as the EL luminous efficiency (or the EL quantum efficiency).

Injected and excited electrons emit light by recombining with holes. This recombination does not necessarily occur with a probability of 100%. Therefore, when organic compounds which each constitute the light emitting layer 1 are compared with each other, the EL luminous efficiencies are compared, thereby making it possible to compare the ratios of the emitted optical energy amount to injected optical energy, and compare synergetic effects about the ratio of the recombination of electrons and holes. Incidentally, by comparing the PL luminous efficiencies, the ratios of the emitted optical energy amount to injected optical energy can be compared. Thus, by comparing both the PL luminous efficiencies and the EL luminous efficiencies and combining the results, it is possible to compare the ratios of the recombination of electrons and holes.

For the PL luminous efficiency, the degree of light emission is preferably as high as possible. The PL luminous efficiency is preferably 20% or more, more preferably 30% or more. The upper limit of the PL luminous efficiency is 100%.

For the EL luminous efficiency, the degree of light emission is preferably as high as possible. The EL luminous efficiency is preferably $1 \times 10^{-3}$% or more, more preferably $5 \times 10^{-3}$% or more. The upper limit of the EL luminous efficiency is 100%.

The light emitting transistor element 10 is characterized by the wavelength of emitted light besides the above. This wavelength is in a visible ray range. The element has a wavelength varied in accordance with the kind of the organic fluorescent substance used, in particular, the pyrene based compound. When organic fluorescent substances having different wavelengths are combined with each other, various colors can be produced. For this reason, about the wavelength of emitted light, the wavelength itself exhibits a characteristic.

The light emitting transistor element 10 is characterized by light emission. Thus, the element preferably has a luminous brightness to a certain extent. This luminous brightness is defined as the light emission amount corresponding to the brightness of an object felt by a person when the person watches the object. This luminous brightness is preferably as high as possible when measured by a photo-counter. The luminous brightness is preferably $1 \times 10^4$ CPS (count per sec) or more, more preferably $1 \times 10^5$ CPS or more, even more preferably $1 \times 10^6$ CPS or more.

The light emitting layer 1 is formed by depositing an organic fluorescent substance or the like that constitute the light emitting layer 1 (or co-depositing a plurality of such substances). It is sufficient if the film thickness of this light emitting layer is at least about 70 nm.

The source electrode 2 and the drain electrode 3 are electrodes for injecting holes and electrons into the light emitting layer 1, and are made of gold (Au), magnesium-gold alloy (MgAu), or the like. The electrodes are formed so as to face each other at a very small interval of, for example, 0.4 to 50 μm. Specifically, for example, as shown in Fig. 6, the source electrode 2 and the drain electrode 3 are formed to have comb tooth shaped regions 2a and 3a, respectively, which are each made of a plurality of comb teeth. The comb teeth which constitute the comb tooth shaped region 2a of the source electrode 2 and the comb teeth which constitute the comb tooth shaped region 3a of the drain electrode 3 are alternately arranged at predetermined intervals, whereby the light emitting transistor element 10 can exhibit the function thereof more effectively.

At this time, the interval between the source electrode 2 and the drain electrode 3, that is, the interval between the comb tooth shaped region 2a and the comb tooth shaped region 3a is preferably 50 μm or less, more preferably 3 μm or less, even more preferably 1 μm or less. If the interval is more than 50 μm, sufficient semi-conductivity cannot be exhibited.

By applying a voltage to the source electrode 2 and the drain electrode 3 in the light emitting transistor element 10, holes and electrons are shifted inside the element and they are recombined in the light emitting layer 1, whereby light can be emitted. At this time, the amounts of the holes and the electrons shifted between the two electrodes across the light emitting layer 1 depend on the voltage applied to the gate electrode 4. Accordingly, by controlling the voltage applied to the gate electrode 4 and its change, it is possible to control the state of electric conduction between the source electrode 2 and the drain electrode 3. Because this light emitting transistor element 10 undergoes P-type driving, a negative voltage for the source electrode 2 is applied to the drain electrode 3 and a negative voltage for the source electrode 2 is applied to the gate electrode 4.

Specifically, by applying a negative voltage for the source electrode 2 to the gate electrode 4, holes in the light emitting layer 1 are attracted toward the gate electrode 4, so that the density of holes in the vicinity of the surface of the insulating film 5 increases. By suitably adjusting the voltage between the source electrode 2 and the drain electrode 3, holes are injected from the source electrode 2 into the light emitting layer 1 according to the intensity of the controlled voltage applied to the gate electrode 4, so that electrons are injected from the drain electrode 3 into the light emitting layer 1. In other words, the source electrode 2 functions as a hole injecting electrode, and the drain electrode 3 functions as an electron injecting electrode. In this way, in the light emitting layer 1, the holes and the electrons are recombined, and light is emitted following this recombination. This light emission state can be turned on or off or the luminous intensity

can be varied by changing the controlled voltage applied to the gate electrode 4.

The theory of such recombination of holes and electrons can be described as follows:

When a negative voltage for the source electrode 2 is applied to the gate electrode 4, in the light emitting layer 1, as illustrated in Fig. 7(a), channel 11 of holes are formed near the interface of the insulating film 2 so that a pinch-off point 12 thereof forms in the vicinity of the drain electrode 3. A high electric field is then formed between the pinch-off point 12 and the drain electrode 3, so that as shown in Fig. 7(b), the energy band is significantly bent. This produces an FN (Fowler-Nordheim) tunnel effect in which electrons in the drain electrode 3 penetrate through the potential barrier between the drain electrode 3 and the light emitting layer 1, so that the electrons are injected into the light emitting layer 1 and recombined with the holes.

The recombination of holes and electrons can also be described on the basis of the following theory besides the FN tunnel effect. As shown in Fig. 7(c), electrons at the HOMO energy level of the organic fluorescent substance in the light emitting layer 1 are excited to the LUMO level thereof by a high electric field. The excited electrons are recombined with holes in the light emitting layer 1. At the same time, electrons are injected from the drain electrode 3 to the HOMO energy level, which is now empty due to the excitation to the LUMO energy level, so that the empty level is filled.

A plurality of such light emitting transistor elements 10 are two-dimensionally arranged on a substrate 20 to form a display device 21. Fig. 8 shows an electric circuit diagram of this display device 21. Specifically, in this display device 21, light emitting transistor elements 10 as described above are each arranged in one of pixels P11, P12, ···,···, P21, P22, ···,···, which are arranged in a matrix form. The light emitting transistor elements 10 in these pixels are selectively caused to emit light and further the luminous intensity (brightness) of the light emitting transistor element 10 in each of the pixels is controlled, whereby two-dimensional display can be attained. The substrate 20 may be, for example, a silicon substrate integrated with the gate electrode 4. In other words, the gate electrode 4 may be made of an electroconductive layer which is an impurity diffusion layer wherein a pattern is formed in a surface of a silicon substrate. As the substrate 20, a glass substrate may be used.

Since each of the light emitting transistor elements 10 undergoes P-type driving, a bias voltage $V_d$ (< 0) is given to its drain electrode 3(D) with the source electrode 2(S) kept at the ground voltage (= 0). To its gate electrode 4(G), a selecting transistor Ts for selecting a pixel and a capacitor C for storing data are connected in parallel.

The selecting transistors Ts in each row of the pixels P11, P12, ···,···,P21, P22, ···, ···, have their gates connected to a common one of the scanning line LS1, LS2, ···,···. The selecting transistors Ts in each column of the pixels P11, P21, ····,···,P12, P22, ···,··· are connected to a common one of the data lines LD1, LD2.... on their side opposite to the respective light emitting transistor elements 10.

From a scanning line driving circuit 61 controlled by a controller 63, scanning driving signals for selecting the pixels P11, P12, ···,···,P21, P22, ···,··· in the respective rows circularly and successively (selecting the plurality of pixels in each row at a time) are given to the scanning lines LS1, LS2, ···,···. In other words, the scanning line driving circuit 22 makes it possible to specify each of the rows successively as a selected row and make the selecting transistors Ts of the plurality of pixels in the selected row electrically conductive at a time, thereby generating a scanning driving signal for cutting off the selecting transistors Ts of the plurality of pixels in the non-selected rows at a time.

On the other hand, signals from a data line driving circuit 23 are inputted into the data lines LD1, LD2, ---,---. Control signals corresponding to image data are inputted from the controller 24 into this data line driving circuit 23. At a timing when the pixels in each of the rows are collectively selected by the scanning line driving circuit 21, the data line driving circuit 23 supplies light emission controlling signals, which correspond to the light emission gradations of the individual pixels in the selected row, to the data lines LD1, LD2, ···,··· in parallel.

In this way, in the individual pixels in the selected row, the light emission controlling signals are given to the gate electrodes 4(G) through the selecting transistors Ts. Thus, the light emitting transistor elements 10 in the pixels emit light having gradations corresponding to the light emission controlling signals (or stop the light emission). Since the light emission controlling signals are kept in the capacitor C, the electric potentials of the gate electrodes 4(G) are kept even after the selected row selected by the scanning line driving circuit 22 is shifted to a different row. As a result, the light emission states of the light emitting transistor elements 10 are kept. Thus, two-dimensional display can be attained.

Description is now made of an organic EL element wherein the above-described pyrene based compound is used.

The organic EL element is an element having a light emitting layer which is capable of transporting holes and electrons, emits light by recombination of the holes and the electrons, and contains the above-mentioned pyrene based compound as a main component, a hole injecting electrode for injecting the holes into this light emitting layer, and an electron injecting electrode for injecting the electrons into the light emitting layer. Specifically, the organic EL element is an element as shown in Fig. 9. This organic EL element 30 is a laminate wherein the following are successively laminated on a substrate 31: a hole injecting electrode layer 32, a hole transporting layer 33, a light emitting layer 34, an electron transporting layer 35, and an electron injecting electrode layer 36. A voltage is applied between the hole injecting electrode

layer 32 and the electron injecting electrode layer 36 by means of a direct current power source 37.

The substrate 31 is a member for supporting the organic EL element 30. This substrate 31 is a transparent substrate such as a glass substrate if the light generated in the light emitting layer 34 has to be delivered to the outside.

The hole injecting electrode layer 32 is a layer to which a positive voltage from the direct current power source 37 is applied. The material thereof is not particularly limited as long as the material has electroconductivity. If the light generated in the light emitting layer 34 has to be delivered to the outside through the substrate 31, it is necessary that this hole injecting electrode layer also have transparency. For such a hole injecting electrode layer, indium tin oxide (ITO) or the like can be used.

The electron injecting electrode layer 36 is a layer to which a negative voltage from the direct current power source 37 is applied. The material thereof is not particularly limited as long as the material has electroconductivity. For example, aluminum is used. If the light generated in the light emitting layer 34 has to be delivered to the outside through the electron injecting electrode layer 36, it is advisable to use, for this electron injecting electrode layer 36, a transparent material such as ITO.

The hole transporting layer 33 is a layer for sending holes generated in the hole injecting electrode layer 32 to the light emitting layer 34. The electron transporting layer is a layer for sending electrons generated in the electron injecting electrode layer 36 to the light emitting layer 34. The hole injecting electrode layer 32 or the electron injecting electrode layer 36 may be laminated directly on the light emitting layer 34. However, from the viewpoint of a problem about the bondability between the two, a layer which has good bondability to both of the hole injecting electrode layer 32 or electron injecting electrode layer 36 and the light emitting layer 34 and which is good in hole- or electron-moving performance is formed as the hole transporting layer 33 or the electron transporting layer 35. For this reason, according to a combination of the material of the hole injecting electrode layer 32 or the electron injecting electrode layer 36 with that of the light emitting layer 34, the hole transporting layer 33 or the electron transporting layer 35 may be omitted.

Examples of the material constituting the hole transporting layer 33 include N,N'-diphenyl-N,N'-bis(3-methylphenyl)-1,1'-biphenyl-4,4'-diamine (TPD). Examples of the material constituting the electron transporting layer 35 include 3-(4-biphenyl)-4-phenyl-5-(4-t-butylphenyl)-1,2,4-triazole (TAZ).

The light emitting layer 34 is a layer wherein the above-mentioned pyrene based compound is used as a main component in the same manner as in the light emitting layer 10 in the above-mentioned light emitting transistor element. If necessary, the above-mentioned secondary components are used together.

As described above, as the hole transporting layer 33 or the electron transporting layer 35, a layer is selected which is good in the hole- or electron-moving performance and in the bondability between the hole injecting electrode 32 or electron injecting electrode 36 and the light emitting layer 34. But if it is difficult to satisfy the two properties simultaneously, a hole injecting layer may be formed between the hole injecting electrode layer 32 and the hole transporting layer 33 or an electron injecting layer may be formed between the electron injecting electrode layer 36 and the electron transporting layer 35. This makes it possible to select a material for the hole transporting layer 33 or the electron transporting layer 35 while attaching more importance to the hole-or electron-moving performance and further select a material for the hole injecting layer or the electron injecting layer while attaching more importance to the bondability. As a result, their materials can be selected from a wider range.

In order for the above-mentioned element to exhibit a function as an organic EL element, it is preferred to use the above-mentioned pyrene based compound as the main component of the organic fluorescent substance constituting the light emitting layer 34, or use Alq3 (tris(8-hydroxyquinolato) or the like as the main component and the pyrene based compound as a dopant.

It is preferred that the difference between the HOMO energy level and the LUMO energy level of the light emitting layer 34, the luminous brightness thereof, the PL luminous brightness thereof, the luminous efficiency thereof and the external luminous efficiency thereof each satisfy a predetermined range. If the pyrene based compound having the above-mentioned individual characteristics is used, it is possible to further improve the individual functions by adding the above-mentioned secondary component, such as the dopant, thereto.

The difference between the HOMO energy level and the LUMO energy level is the same as in the case of the light emitting transistor element.

The luminous brightness is defined as the light emission amount corresponding to the brightness of an object felt by a person who watches the object. The luminous brightness is preferably as high as possible. The luminous brightness can be measured by use of an Si photodiode. According to this method, the luminous brightness ($cd/m^2$) generated changes with the current (or voltage) applied. If a current of e.g. 10 $mA/cm^2$ (voltage of 6.0 V) is applied, the luminous brightness should be 50 $cd/m^2$ or more, preferably 75 $cd/m^2$ or more, more preferably 100 $cd/m^2$ or more.

If a current of 100 $mA/cm^2$ (voltage of 8.0 V) is applied, the luminous brightness should be 600 $cd/m^2$ or more, preferably 1000 $cd/m^2$ or more, more preferably 2000 $cd/m^2$ or more.

The PL luminous efficiency is as described above. The external luminous efficiency means the luminous efficiency observed from outside. It is said that the upper limit of this external luminous efficiency is 5% for ordinarily used fluorescent colorants.

For organic EL elements, it is particularly necessary that the degree of light emission be high. The PL luminous efficiency is preferably 80% or more, more preferably 85% or more. The upper limit of the PL luminous efficiency is 100%. The external luminous efficiency should be 1% or more, preferably 1.4% or more, more preferably 2% or more. It is said that the upper limit of the EL luminous efficiency is 5%.

The organic EL element 30 is characterized by the wavelength of emitted light besides the above. This wavelength is in a visible ray range. The element has a wavelength varied in accordance with the kind of the used organic fluorescent substance, in particular, the above-mentioned pyrene based compound. By combining organic fluorescent substances having different wavelengths with each other, various colors can be expressed. For this reason, the wavelength itself of emitted light exhibits a characteristic.

The organic EL element 30 can be formed by forming the hole injecting electrode layer 32 on the substrate 31 and forming, thereon, the individual layers successively by vacuum evaporation. It is sufficient if the film thickness of the light emitting layer 34 in the resultant organic EL element 30 is about 10 to 100 nm, and if the film thickness of the hole transporting layer 33 or the electron transporting layer 35 is about 10 to 50 nm. The film thickness of the hole injecting electrode layer 32 or the electron injecting electrode layer 36 will be sufficient if it is bout 5 to 30 nm.

The operation of the organic EL element 30 is as follows: First, a voltage is applied to the hole injecting electrode layer 32 and the electron injecting electrode layer 36 by means of the direct current power source 37. Holes generated in the hole injecting electrode layer 32 are sent through the hole transporting layer 33 to the light emitting layer 34. Electrons generated in the electron injecting electrode layer 36 are sent through the electron transporting layer 35 to the light emitting layer 34. In the light emitting layer 34, the holes and the electrons are recombined to emit light.

A plurality of the organic EL elements 30 are two-dimensionally arranged on a substrate 40 to form a display device. As an example of this display device 41, an example of the electric circuit diagram of a passive type display device 41 is shown in Fig. 10.

This is a device wherein scanning lines (LS1', LS2', ... ...) and data lines (LD1', LD2', ......) are arranged in a lattice form on the substrate 40. At each of intersection points therebetween, one of the organic EL elements 30 is arranged. Specifically, the organic EL element (1, 1) in row 1, column 1 has one end thereof connected to the scanning line in row 1 and the other end thereof connected to the data line column 1. The organic EL element (j, i) in row j, column I has one end thereof connected to the scanning line in row j and the other end thereof connected to the data line in column i.

When the data line i is set at a high level and the scanning line j is set at a low level, an electric current flows into the organic EL element (j, i) so that the element emits light. By adjusting the period during which the current flows, the gradation of light emission can be controlled. For example, in a scanning line driving circuit 42, the scanning lines corresponding to the rows in which light is to be emitted are selected and set to a low level (for example, 0 V), and the scanning lines corresponding to the rows in which light is not to be emitted are set to a high level. Also, from a data line driving circuit 43, pulse-modulated data signals are supplied as high level data to the data lines for a predetermined period of time according to the gradation of light emission. This makes it possible to cause the selected organic EL elements to emit light so as to display an image.

By changing over the selected scanning lines in the scanning line driving circuit 42, the selected EL elements emit light in turn, so that images change over.

EXAMPLES

The present invention will be more specifically described by way of examples and comparative examples described below. First, the pyrene based compound producing process will be described.

(Production Example 1) [Production of triphenyldodecylpyrene] [Synthesis of a starting material]

[Chemical formula 8]

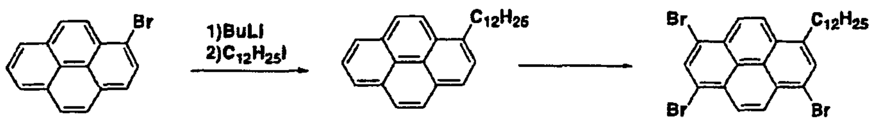


<2>

In accordance with the above-mentioned reaction formula <2>, a raw material was first synthesized. That is,

a 200 ml side-arm flask was fitted with a dropping funnel, a low-temperature thermometer and a cooling tube, and was dried under a reduced pressure and then purged with nitrogen. The flask was charged with 7.0 g of 1-bromopyrene (reagent made by Tokyo Kasei Kogyo Co., Ltd.) and 70 ml of dried THF, and the mixture was stirred in a dry ice/acetone bath at -70°C or lower. 9.8 ml of a 2.6 M solution of butyllithium in hexane (reagent made by Kanto Chemical Co., Inc.) was dropwise added thereto for 20 minutes. The mixture was then stirred at 70°C for 1 hour. Thereafter, 6.4 ml of dodecyl iodide (reagent made by Aldrich Co.) was dropwise added thereto, and the mixture was stirred at 60°C for 1 hour, and then the cooling bath was removed to raise the temperature to room temperature in 1 hour.

The reaction solution was concentrated in an evaporator, and chloroform and pure water were added to the residue to separate the solution into phases. The water phase was extracted with chloroform. The organic phase was dried over anhydrous magnesium sulfate, and concentrated.
Hexane was added to the collected residue, and the precipitation was collected by suction filtration to yield 5.9 g of dodecylpyrene (yield: 64%).

Subsequently, 4.4 g of dodecylpyrene was put thereinto, and the system was purged with nitrogen. Thereafter, 30 ml of DMF (reagent made by Junsei Chemical Co., Ltd.) was added, and the mixture was heated and stirred in an oil bath at 70°C to prepare a homogenous solution. Into 20 ml of the thus obtained DMF, 5.3 g of N-bromosuccinimide (NBS: reagent made by Tokyo Kasei Kogyo Co., Ltd.) was dissolved, and the resultant was dropwise added thereto from the dropping funnel. At an internal temperature of 70°C, the solution was stirred for 6 hours. The solution was cooled, and then the thus obtained precipitation was collected by suction filtration, and washed with methanol to remove high-polarity components and DMF Thereafter, the resultant was dried under a reduced pressure to yield 4.5 g of a solid (yield: 71%). From FAB-MS, a result of m/z = 606 was obtained, so that this component was identified as 1-dodecyl-3,6,8-tribromopyrene.

[Production of triphenyldodecylpyrene]

[Chemical formula 9]

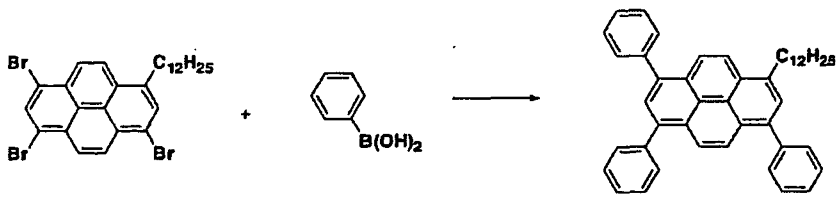

<3>

In accordance with the above-mentioned reaction formula (3), triphenyldodecylpyrene ((4-5) in Fig. 2(a)) was produced. That is, a 500 ml four-necked flask equipped with a reflux condenser tube, a three-way cock, and a thermometer was charged with 2.5 g of 1-dodecyl-3,6,8-tribromopyrene, as described above, 3.3 g of phenylboric acid (reagent made by Aldrich Co.), 17.5 g of cesium carbonate, 180 ml of toluene, 80 ml of ethanol, and 40 ml of pure water. The pressure in the system was reduced and the system was purged with nitrogen. This cycle was repeated five times. Thereafter, as an operation for degassing, the inside of the solution was bubbled with nitrogen. Next, 0.3 g of tetrakistriphenylphosphine palladium (0), as described above, was added thereto, and then the solution was heated and stirred in an oil bath at 80°C for 8 hours.

The organic phase was separated, and the water phase was extracted with 50 ml of chloroform. The collected organic phase was washed with 300 ml of pure water, dried over anhydrous magnesium sulfate, and concentrated with an evaporator. Hexane was added to the residue, and the precipitation was collected by suction filtration. Palladium mixed therewith was removed by column chromatography (silica gel, chloroform), and the solid precipitated by the concentration was washed with methanol, and collected. The collected solid was purified by GPC, so as to yield 530 g of yellow crystals. From $^1$H-NMR, the crystals were identified as 1-dodecyl-3,6,8-triphenylpyrene (yield: 21%).

$^1$H NMR($CDCl_3$) δ8.26 (s)1H,δ8.25 (s)1H,δ8.08 (s)1H,δ8.08 (s)1H,δ7.97 (s)1H,δ7.87 (s)1H,δ7.75-7.41 (m)15H, δ3.34 (t)2H,δ1.87 (m)2H,δ1.50 (m)2H,δ1.45-1.15 (m)16H,δ0.86 (t)3H

(Production Example 2) [Production of tri(4-biphenyl)-dodecylpyrene]

[Chemical formula 10]

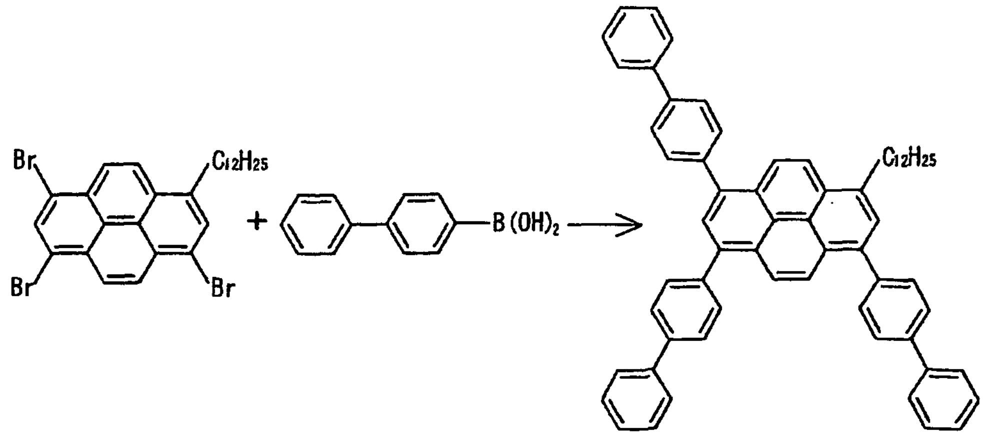

(4)

In accordance with the above-mentioned reaction formula (4), tri(4-biphenyl)-dodecylpyrene ((4-7) in Fig. 2(a)) was produced. That is, a 500 ml three-necked flask equipped with a reflux condenser tube, a three-way cock, and a thermometer was fitted with a reflux condenser tube, a three-way cock connected to a nitrogen line, and a thermometer. Into this system were charged 4.00 g of 1-dodecyl-3,6,8-tribromopyrene, as described above, 6.54 g of 4-biphenylboric acid (reagent made by Aldrich Co.), 7.00 g of sodium carbonate (reagent made by Kanto Chemical Co., Inc.), 150 ml of toluene (reagent made by Junsei Chemical Co., Ltd.), 70 ml of ethanol (reagent made by Junsei Chemical Co., Ltd.), and 30 ml of pure water. The solution was stirred at room temperature. The system was degassed by reducing the pressure and purged with nitrogen. This was repeated five times to purge the system with nitrogen. Thereafter, the inside of the reaction solution was bubbled with nitrogen for 30 minutes. Next, 0.48 g of tetrakistriphenylphosphine palladium (0), as described above, was added thereto, and then the solution was refluxed in an oil bath at 80°C for 10 hours. Thereafter, the resultant was left at rest overnight in the atmosphere of nitrogen.
The solid precipitated by cooling was collected by suction filtration, and recrystallized from toluene to yield yellow crystals. From $^1$H-NMR, the yellow crystals were identified as the intended 1-3,6,8-tri(4-biphenyl)-8-dodecylpyrene (yield: 64%).
$^1$H NMR($CDCl_3$) δ8.34 (s) 1H,δ8.33 (s) 1H,δ8.21 (s) 2H,δ8.08 (s) 1H,δ7.94 (s) 1H,δ7.87-7.67 (m) 18H,δ7.58-7.47 (m) 6H, δ7.45-7.35 (m) 3H,δ3.38 (t) 2H,δ1.92 (m) 2H,δ1.55 (m) 2H,δ1.47 -1.17(m) 18H,δ0.86 (t) 3H

(Production Example 3) [Production of tri(2-naphthyl)dodecylpyrene]

[Chemical formula 11]

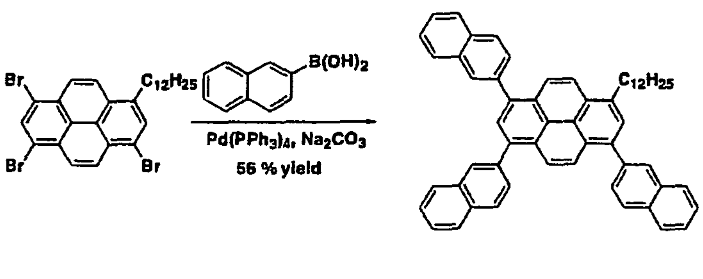


<5>

A 500 ml four-necked flask equipped with a reflux condenser tube, a three-way cock, and a, thermometer was charged with 3.0 g of 1-dodecyl-3,6,8-tribromopyrene, 4.4 g of 2-naphthylboric acid (reagent made by Aldrich Co.), 5.3 g of sodium carbonate (reagent made by Kanto Chemical Co., Inc.), 120 ml of toluene (reagent made by Junsei Chemical

Co., Ltd.), 60 ml of ethanol (reagent made by Junsei Chemical Co., Ltd.), and 10 ml of pure water. The system was purged with nitrogen. Thereafter, 0.4 g of tetrakistriphenylphosphine palladium (0) (reagent made by Tokyo Kasei Kogyo Co., Ltd.) was added thereto, and then the solution was heated and stirred in an oil bath at 80°C for 9 hours.
The organic phase was separated, and the water phase was extracted twice with 50 ml of $CHCl_3$. The collected organic phase was washed with 200 ml of pure water, dehydrated over anhydrous magnesium sulfate, filtered and then concentrated in an evaporator. The resultant solid was subjected to column chromatography ($SiO_2$, $CHCl_3$) to remove high-polarity components. Thereafter, the effluent was concentrated, and the resultant solid was purified by GPC to yield 2.1 g of a yellow solid.
From the mass through ionization by FAB, M-1 was found out at 748 in connection with the intended molecular weight of 749. Also, from 1H NMR, this substance was identified as 1-dodecyl-3,6,8-tri(2-naphthyl)-pyrene.

$^1$H NMR (270MHz,$CDCl_3$) 8.32ppm (s,2H),8.20-8.05 (m,6H),8.04-7.76 (m,13H),7.61-7.49 (m,6H),3.38 (t,2H, J=7.56),1.98-1.85 (m,2H),1.58-1.45 (m,2H),1.44-1.18 (m,16H),0.85 (t,3H,J=6.75))

Mass(MALDI-TOF):Obs.m/z=748 ($M^+$-1),593,580,465;Calc. for C58H52,749.05.

(Production Example 4) [Production of tri(3-pyridyl)dodecylpyrene]

[Chemical formula 12]

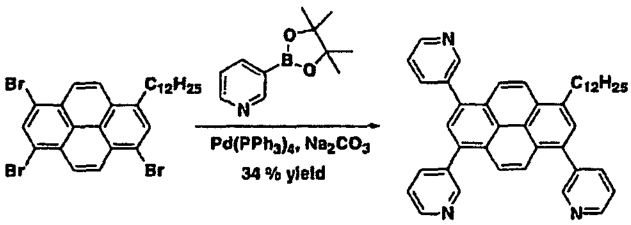


<6>

A 500 ml four-necked flask equipped with a reflux condenser tube, a three-way cock, and a thermometer was charged with 3.3 g of 1-dodecyl-3,6,8-tribromopyrene, 5.7 g of a pinacol ester of 2-pyridylboric acid (reagent made by Aldrich Co.), 5.7 g of sodium carbonate (reagent made by Kanto Chemical Co., Inc.), 120 ml of toluene (reagent made by Junsei Chemical Co., Ltd.), 60 ml of ethanol (reagent made by Junsei Chemical Co., Ltd.), and 30 ml of pure water. The system was purged with nitrogen. Thereafter, 0.3 g of tetrakistriphenylphosphine palladium (0) (reagent made by Tokyo Kasei Kogyo Co., Ltd.) was added thereto, and then the solution was heated and stirred in an oil bath at 80°C for 7 hours.
The organic phase was separated, and the water phase was extracted with 50 ml of $CHCl_3$. The collected organic phase was washed with 100 ml of pure water, dehydrated over anhydrous magnesium sulfate, filtered and then concentrated in an evaporator. Hexane was added to the resultant residue, and the solution was refluxed and thermally filtered to remove remaining Pd and other inorganic salts. The precipitated solid was collected by suction filtration, and this solid was again recrystallized from hexane. The collected solid was subjected to column chromatography ($SiO_2$, $CHCl_3$-acetone) to remove salts. Thereafter, the effluent was purified by GPC to yield 1.1 g of a yellow solid. From FAB mass spectrometry (M+1: 602) and $^1$H NMR, the solid was identified as 1-dodecyl-3,6,8-tri(3-pyridyl)pyrene

·$^1$H NMR(270MHz,$CDCl_3$) 8.95-8.88 (m,3H),8.79-8.71 (m,3H),8.37 (d,1H,J=9.72),8.19(d,1H,J=9.45),8.10-7.88 (m,7H),7.57-7.47(m,3H),3.38 (t,3H,J=7.56),1.95-1.82 (m,2H),1.57-1.45 (m,2H),1.44-1.18 (m,16H),0.86 (t, 3H, J=6.48)

·Mass(MALDI-TOF):Obs.m/z=602 ($M^+$+1),446;Calc. for C43H43N3,601.83.

(Production Example 5) [Production of trinaphthylpyrene]

[Production of 3,6,8-tribromopyrene]

[Chemical formula 13]

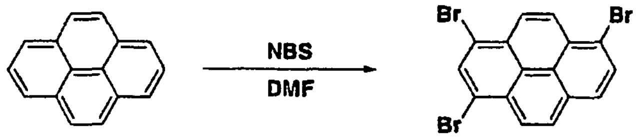


<7>

To a solution of 5.0 g of pyrene (reagent made by Aldrich Co.) in 80 ml of DMF was dropwise and slowly added a solution of 26.8 g of NBS (reagent made by Tokyo Kasei Kogyo Co., Ltd.) in 100 ml of DMF, and then the reaction temperature was raised to 130°C and the solution was heated and stirred.
The precipitated solid was collected by suction filtration, washed with $CHCl_3$, and then recrystallized from o-dichlorobenzene to yield 9.7 g of needle crystals. From FAB mass spectrometry, the molecular weight of the needle crystals was 435, and the crystals were identified as 3, 6, 8-tribromopyrene.

[Production of trinaphthylpyrene]

[Chemical formula 14]

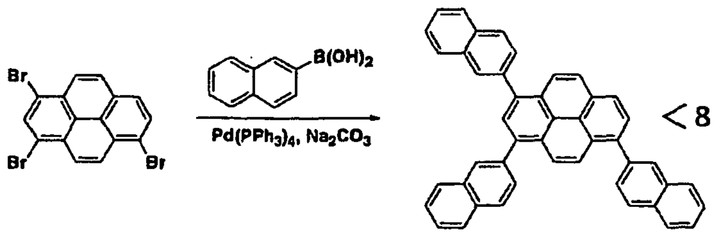


<8>

To 4.3 g of 3,6,8-tribromopyrene, 8.6 g of 2-naphthylboric acid (reagent made by Aldrich Co.), and 6.0 g of cesium carbonate (reagent made by Kanto Chemical Co., Inc.) were added 200 ml of toluene (reagent made by Junsei Chemical Co., Ltd.), 25 ml of ethanol (reagent made by Junsei Chemical Co., Ltd.), and 25 ml of pure water. The system was purged with nitrogen, and then 1.0 g of tetrakistriphenylphosphine palladium (0) (reagent made by Tokyo Kasei Kogyo Co., Ltd.) was added thereto. The solution was then heated and refluxed for 9 hours.
The solvent was distilled off, and 100 ml of water was added to the resultant. The resultant was extracted twice with 50 ml of $CH_2Cl_2$. Anhydrous sodium sulfate was added to the resultant dichloromethane solution to dehydrate the solution. The solution was filtered, and concentrated in an evaporator. The resultant solid was recrystallized from toluene, and further purified by GPC to yield 1 g of a yellow solid.
Mass(MALDI-TOF):Obs.m/z=580 ($M^+$);Calc. for C46H28,580.73.

(Production Example 6) [Production of triphenylpyrene]

[Production of tribromopyrene]

[Chemical formula 15]

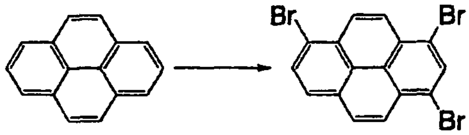

<9>

A 1000 mL four-necked flask was fitted with a dropping funnel, a reflux condenser tube, a three-way cock and a thermometer, and was purged with nitrogen. The flask was then charged with 20.0 g of pyrene (reagent made by Aldrich Co.) and 200 ml of DMF, and then was again purged with nitrogen. The system was heated at an internal temperature of 70°C to dissolve pyrene. Into 400 ml of DMF was dissolved 108.9 g of NBS (reagent made by Tokyo Kasei Kogyo Co., Ltd.), and this was dropwise added thereto from the dropping funnel over 20 minutes. After the end of the addition, the heating temperature was raised from 70°C to 130°C. At this temperature, reaction was conducted for 8 hours. The resultant was cooled, and then the resultant solid was collected by suction filtration. This was washed with ethanol to yield 39.3 g of a crude product of 1,3,6-tribromopyrene (yield: 92.3%).
The resultant crude product was recrystallized from o-dichlorobenzene having a volume 15 times as much as the volume of the sample to collect light gray needle crystals. From LC analysis, the purity of the collected product was 91% (as other components, dibromopyrene and tetrabromopyrene were found).

[Production of triphenylpyrene]

[Chemical formula 16]

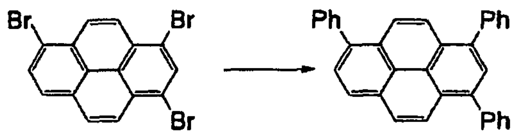

<10>

A 1000 ml four-necked flask equipped with a stirring motor, a reflux condenser tube, and a three-way cock connected to a nitrogen line was charged with 20.1 g of 1,3,6-tribromopyrene, the LC purity of which was 91%, 23.9 g of phenylboric acid (reagent made by Aldrich Co., purity: 95%), 39.4 g of sodium carbonate (reagent made by Kanto Chemical Co., Inc.), 400 ml of toluene (reagent made by Junsei Chemical Co., Ltd.), 200 ml of ethanol (reagent made by Junsei Chemical Co., Ltd.), and 80 ml of desalted water. While the solution was stirred, the pressure in the reactor was reduced. Subsequently, the reactor was purged with nitrogen, and this operation was performed twice. Thereafter, the reaction solution was bubbled with nitrogen to degas the solution. Then, 2.8 g of tetrakistriphenylphosphine palladium (0) was added thereto, and the resultant was heated and stirred in an oil bath at 80°C for 9 hours.
After the reaction, 200 ml of desalted water was added thereto, and the reaction solution was subjected to suction filtration. To the filtrate was added 200 ml of toluene to separate the solution into phases. Furthermore, the water phase was extracted with 200 ml of toluene. The organic phases were combined, and the resultant organic phase was dehydrated over anhydrous magnesium sulfate and concentrated in an evaporator. The resultant crude product was washed with acetonitrile to yield 15.1 g of 1,3,6-triphenylpyrene (yield: 84.9%). From LC analysis, the collected product had a purity of 88.2%. In the specification, "-Ph" in any chemical formula represents a phenyl group.

(Production Example 7) [Production of triphenylbromopyrene]

[Chemical formula 17]

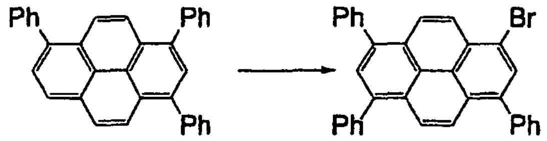

<11>

A 200 ml three-necked flask was fitted with a reflux condenser tube, a three-way cock connected to a nitrogen line, a dropping funnel, and a thermometer, and the inside of the reactor was purged with nitrogen. The flask was then charged with 6.2 g of 1,3,6-triphenylpyrene, and 80 ml of DMF (reagent made by Junsei Chemical Co., Ltd.), and then the solution was stirred at an internal temperature of 90°C. A solution formed by dissolving 2.6 g of NBS into 20 ml of DMF was dropwise added thereto from the dropping funnel for 5 minutes. The solution was stirred for 1 hour while the temperature was kept at 90°C.
The resultant was cooled, and then the precipitated solid was collected by suction filtration. The crude product was washed with methanol. The collected crude product was recrystallized from toluene having a volume about three times the volume of the sample to yield 3.8g of 1-bromo-3,6,8 triphenylpyrene (yield: 52.2%). It was analyzed by $^1$H NMR. As a result, the purity was 90%.

(Production Example 7-1) [Production of p-phenylenebis(3,6,8-triphenylpyrene)]

[Chemical formula 18]

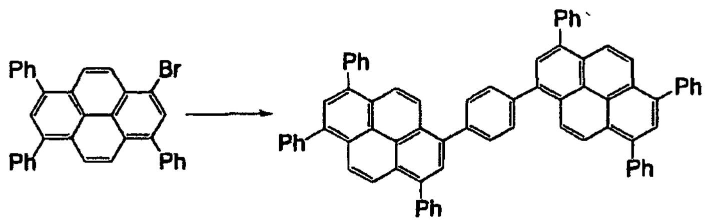

<12>

A 200 ml three-necked flask equipped with a reflux condenser tube, a three-way cock connected to a nitrogen line and a thermometer was charged with 3.9 g of 1-bromo-3,6,8-triphenylpyrene, 0.37 g of p-phenylenebisboric acid (reagent made by Aldrich Co.), 1.2 g of sodium carbonate (reagent made by Kanto Chemical Co., Inc.), 50 ml of toluene (reagent made by Junsei Chemical Co., Ltd.), 20 ml of ethanol (reagent made by Junsei Chemical Co., Ltd.), and 5 ml of desalted water. The pressure in the system was reduced, and the system was purged with nitrogen. This operation was repeated five times. Furthermore, nitrogen was passed into the reaction solution for 30 minutes to degas the solution. Then, 0.32 g of tetrakistriphenylphosphine palladium (0) was added thereto, and the resultant was heated and stirred in an oil bath at 80°C for 11 hours.
The system was cooled, and the precipitated solid was collected by suction filtration, and washed with methanol to yield 1.7 g of a white solid. From mass spectrometry through DEI ionization, a result of m/Z = 934 was obtained, and this was identified as the intended p-phenylenebis-(3,6,8-triphenylpyrene) (yielded amount: 1.72 g, yield: 83%).

Mass(DEI)Obs.m/Z=934 ($M^+$),Calc. for $C_{74}H_{46}$

(Production Example 7-2) [Production of 4,4'-biphenylenebis(3,6,8-triphenylenepyrene)]

[Chemical formula 19]

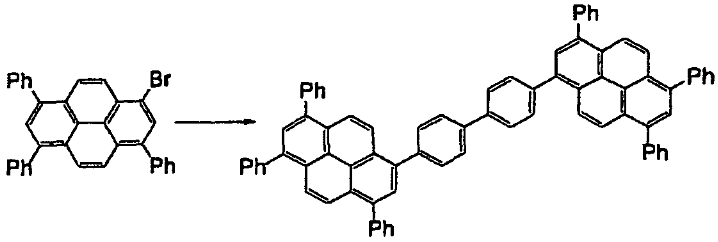

<1 3>

A 200 ml three-necked flask equipped with a reflux condenser tube, a three-way cock connected to a nitrogen line and a thermometer was charged with 9.8 g of 1-bromo-3,6,8-triphenylpyrene, 1.3 g of 4,4'-biphenylenebisboric acid (Lancaster reagent), 2.9 g of sodium carbonate (reagent made by Kanto Chemical Co., Inc.), 100 ml of toluene (reagent made by Junsei Chemical Co., Ltd.), 30 ml of ethanol (reagent made by Junsei Chemical Co., Ltd.), and 10 ml of desalted water. The pressure in the system was reduced, and the system was purged with nitrogen. This operation was repeated five times. Furthermore, nitrogen was passed into the reaction solution for 30 minutes to degas the solution. Then, 0.7 g of tetrakistriphenylphosphine palladium (0) was added thereto, and the resultant was heated and stirred in an oil bath at 80°C for 12 hours.
The system was cooled, and the precipitated solid was collected by suction filtration, and washed successively with desalted water and methanol to yield a crude product. The collected crude product was recrystallized from toluene having a volume 30 times the volume of the sample to yield 2.5 g of 4,4'-biphenylenebis(3,6,8-triphenylpyrene) (yielded amount: 3.4 g, yield: 65%). From LC analysis, the purity of the collected product was 99.4%.
$^1H$ NMR(400MHz,$CDCl_3$) δ8.30 (d,J=10.00,2H),8.22 (d,J=9.60,2H),8.18 (s,4H),8.08 (s,2H),8.02 (s,2H),7.89 (dd,J=8.40,4H),7.81 (dd,J=8.40,4H),7.72-7.66 (m,12H),7.58-7.51 (m, 12H), 7.50-7.42 (m,6H)
Mass(DEI)Obs.m/Z=1010 ($M^+$),Calc. for $C_{80}H_{50}$

(Production Example 7-3) [Production of 9,9'-dihexyl-2,7-fluorenebis(3,6,8-triphenylpyrene)]

[Chemical formula 20]

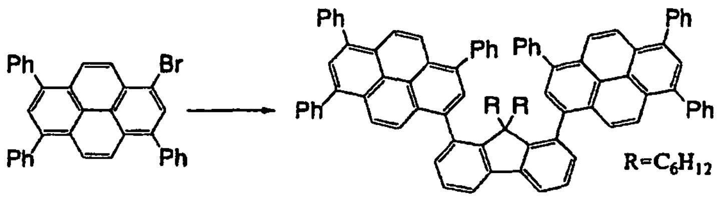

<1 4>

A 100 ml three-necked flask equipped with a reflux condenser tube, a three-way cock connected to a nitrogen line and a thermometer was charged with 3.0 g of 1-bromo-3,6,8-triphenylpyrene, 0.7 g of 9,9'-dihexyl-2,7-fluorenebisboric acid (reagent made by Aldrich Co.), 0.9 g of sodium carbonate (reagent made by Kanto Chemical Co., Inc.), 40 ml of toluene (reagent made by Junsei Chemical Co., Ltd.), 15 ml of ethanol (reagent made by Junsei Chemical Co., Ltd.), and 4 ml of desalted water. The pressure in the system was reduced, and the system was purged with nitrogen. This operation was repeated five times. Furthermore, nitrogen was passed into the reaction solution for 30 minutes to degas the solution. Then, 0.3 g of tetrakistriphenylphosphine palladium (0) was added thereto, and the resultant was heated and stirred in an oil bath at 80°C for 12 hours.
To the reaction mixture was added 40 ml of desalted water to separate the solution into phases. Furthermore, the water phase was extracted twice with 30 ml of chloroform. The organic phases were combined, and the resultant phase was dehydrated over anhydrous magnesium sulfate, and concentrated to yield a crude product. The collected crude product

was purified by GPC to yield 9,9'-dihexyl-2,7-fluorenebis(3,6,8-triphenylpyrene) (yielded amount: 1.7 g, yield: 79%).

$^1$H NMR(400MHz,$CDCl_3$) δ8.21 (d,J=10.00,2H),8.19-8.16 (m,6H),8.11 (s,2H),8.02 (s,2H),7.78-7.65 (m, 14H), 7.60-7.51 (m,12H),7.50-7.42 (m,6H),2.12-2.05 (m,4H),1.30-1.22 (m,4H)1.08 (s,12H),0.67 (t,J=6.80,6H)

Mass(DEI)Obs.m/Z=1190 ($M^+$),Calc. for $C_{93}H_{74}$

(Production Example 8) [Production of 3,6,8-triphenylpyreneboric acid]

[Chemical formula 21]

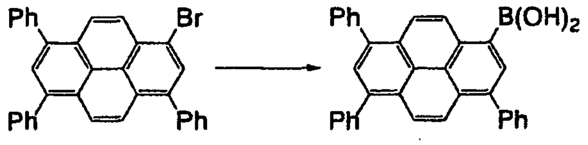

<15>

Using a vacuum pump and by heating, with a heat gun, a 300 ml three-necked flask in which is received a stirrer and which is equipped with a low-temperature thermometer, a dropping funnel, and a three-way cock connected to a nitrogen line, the inside of the reactor was dried and purged with nitrogen. Into this reactor was put 7.6 g of triphenylmonobromopyrene with a nitrogen flow to purge the inside again with nitrogen. Then, after adding 200 ml of dry THF (reagent made by Kanto Chemical Co., Inc.), a dry ice/acetone bath was used to cool the reaction solution to -78°C. Thereafter, 12 ml of a n-butyllithium/hexane solution (1.6 N, reagent, Kanto Chemical Co., Inc.) was dropwise added thereto for 15 minutes. The solution was stirred for 2 hours while the temperature was kept as it was. Thereto was dropwise added 5 ml of trimethyl borate (reagent, Kanto Chemical Co., Inc.) from a syringe. Thereafter, the solution was continuously stirred for 1 hour. The cooling bath was removed to raise the temperature to room temperature. The reaction solution was concentrated in an evaporator, and the residue was separated into phases with 200 ml of chloroform and 100 ml of 1 N HCl. The organic phase was collected and concentrated. The resultant crude product was washed with methanol to yield yellow crystals (yielded amount: 6.3 g, yield: 87%).

(Production Example 8-1) [Production of 2,2'-bipyridyl-6,6'-bis(3,6,8-triphenylpyrene)]

[Chemical formula 22]

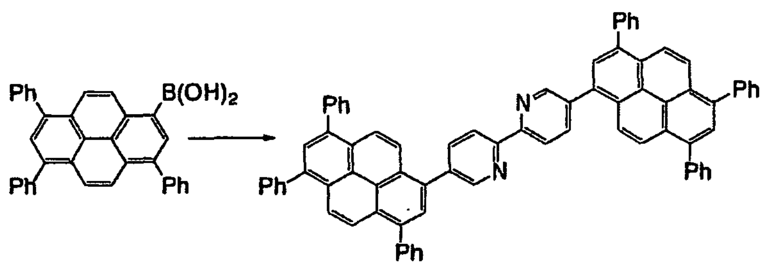

<16>

A 300 ml three-necked flask equipped with a reflux condenser tube, a three-way cock connected to a nitrogen line and a thermometer was charged with 2.7 g of 3,6,8-triphenylpyreneboric acid, 0.6 g of 4,4'-dibromopyridine, 1.2 g of sodium carbonate (reagent made by Kanto Chemical Co., Inc.), 50 ml of toluene (reagent made by Junsei Chemical Co., Ltd.), 20 ml of ethanol (reagent made by Junsei Chemical Co., Ltd.), and 10 ml of desalted water. The pressure in the system was reduced, and the system was purged with nitrogen. This operation was repeated five times. Furthermore, nitrogen was passed into the reaction solution for 30 minutes to degas the solution. Then, 0.2 g of tetrakistriphenylphosphine palladium (0) was added thereto, and the resultant was heated and stirred in an oil bath at 80°C for 10 hours.

The system was cooled, and the precipitated solid was collected by suction filtration, and washed with methanol to yield a yellow solid (yielded amount: 1.7 g, yield: 81%).

Mass(DEI)Obs.m/Z=1012 ($M^+$),Calc. for $C_{78}H_{48}N_2$

(Production Example 8-2) [Production of 2,2'-biphenylenebis-(3,6,8-triphenylpyrene)]

[Chemical formula 23]

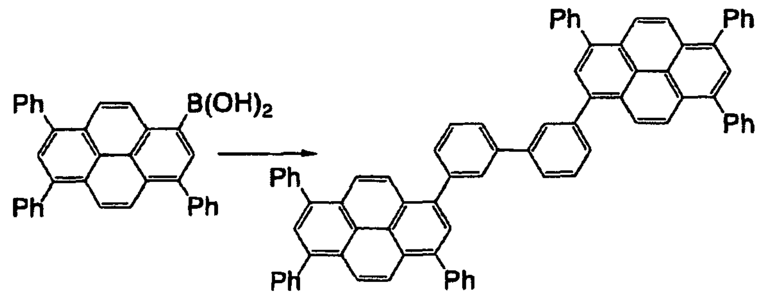

<17>

A 200 ml three-necked flask equipped with a reflux condenser tube, a three-way cock connected to a nitrogen line and a thermometer was charged with 4.3 g of 3,6,8-triphenylpyreneboric acid, 0.9 g of 3,3'-dibromobiphenyl, 1.9 g of sodium carbonate (reagent made by Kanto Chemical Co., Inc.), 60 ml of toluene (reagent made by Junsei Chemical Co., Ltd.), 4 ml of ethanol (reagent made by Junsei Chemical Co., Ltd.), and 40 ml of desalted water. Nitrogen was passed into the reaction solution for 40 minutes. Then, 0.2 g of tetrakistriphenylphosphine palladium (0) was added thereto, and the resultant was heated and stirred in an oil bath at 80°C for 10 hours.

The precipitated solid was collected by suction filtration, and washed with toluene, THF and DMF to yield a yellow solid (yielded amount: 2.5 g, yield: 82%).

Mass(DEI)Obs.m/Z=1010 ($M^+$),Calc. for $C_{78}H_{48}N_2$

(Production Example 9) [Production of 4,4'-biphenylenebis-(3,6,8-tri-m-tolylpyrene)]

[Chemical formula 24]

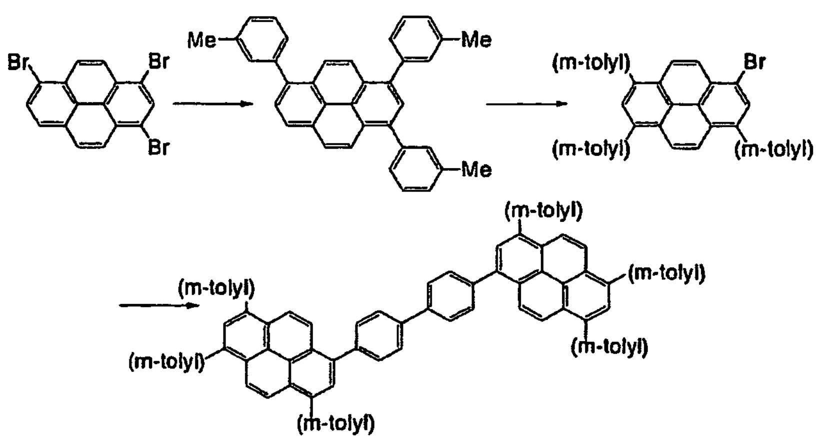

<18>

A 300 ml three-necked flask equipped with a reflux condenser tube, a three-way cock connected to a nitrogen line and a thermometer was charged with 5.7 g of 1,3,6-tribromopyrene, the LC purity of which was 94%, 8.0 g of m-tolylboric acid (reagent made by Aldrich), 12.4 g of sodium carbonate (reagent made by Kanto Chemical Co., Inc.), 150 ml of toluene (reagent made by Junsei Chemical Co., Ltd.), 50 ml of ethanol (reagent made by Junsei Chemical Co., Ltd.), and 15 ml of desalted water. The pressure in the system was reduced, and the system was purged with nitrogen. This operation was repeated five times. Furthermore, nitrogen was passed into the reaction solution for 30 minutes to degas the solution. Then, 1.0 g of tetrakistriphenylphosphine palladium (0) was added thereto, and the resultant was heated and stirred in an oil bath at 80°C for 11 hours.
The reaction solution was cooled, and then 150 ml of desalted water was added to the reaction solution to separate the solution into phases. Furthermore, the water phase was extracted twice with 100 ml of toluene. The organic phases combined with each other were dried over anhydrous magnesium sulfate, and concentrated with an evaporator. The collected residue was subjected to desalting treatment by column chromatography (silica gel, $CHCl_3$) to yield 1,3,6-tris (m-tolyl)pyrene. The LC purity was 94%, and the hygroscopicity was high; accordingly, the yielded amount was larger than the theoretical yielded amount even after the product was dried (yielded amount: 5.9 g, yield: 102%). In the specification, "-Me" represents a methyl group, and "-m-tolyl" represents a m-tolyl group.

$^1H$ NMR(400MHz,$CDCl_3$) δ8.24 (d,1H,J=9.20),8.20 (d,1 H,J=8.40), 8.15 (d,2H,J=3.20),8.05 (d,1H,J=9.20), 7.99 (d,1H,J=8.00),7.98 (s,1H),7.51-7.35 (m,9H),7.34-7.23 (m,3H),2.49 (s,3H),2,47 (s,3H)2.45 (s,3H)

Next, a 200 ml three-necked flask equipped with a dropping funnel and a three-way cock connected to a nitrogen line was charged with 5.2 g of 1,3,6-tris(m-tolyl)pyrene, the LC purity of which was 94%, and 70 ml of DMF (reagent made by Junsei Chemical Co., Ltd.), and then the solution was stirred at room temperature in the atmosphere of nitrogen. To this solution was dropwise added a solution formed by dissolving 2.0g of N-bromosuccinimide (reagent made by Tokyo Kasei Kogyo Co., Ltd., purity: 98%) into 30 ml of DMF, as described above, for 10 minutes. In the atmosphere of nitrogen, the solution was stirred at room temperature for 6 hours. It was confirmed that the starting materials disappeared. Thereafter, 50 ml of desalted water and 50 ml of MeOH (reagent made by Junsei Chemical Co., Ltd.) were added thereto to precipitate the intended product. The collected solid was washed with MeOH to yield a crude product. The resultant crude product was purified by GPC to yield 3,6,8-tris(m-tolyl)-1-bromopyrene (yielded amount: 3.7 g, yield: 60%).

$^1H$ NMR(400MHz,$CDCl_3$) δ8.44 (d,1H,J=9.60),8.34 (d,1H,J=9.60),8.25(s,1H),8.18(d,1H,J=9.60),8.08(d,1H, J=9.60),8.01 (s, 1H), 7.50-7.38 (m,9H),7.32-7.26 (m,3H),2.49 (s,3H),2,47 (s,3H)2.45 (s,3H)

Next, a 100 ml three-necked flask equipped with a reflux condenser tube, a three-way cock connected to a nitrogen line and a thermometer was charged with 3.0 g of 3,6,8-tris(m-tolyl)-1-bromopyrene, 0.5 g of 4,4'-biphenylbisboric acid (reagent made by Aldrich), 0.9 g of sodium carbonate (reagent made by Kanto Chemical Co., Inc.), 30 ml of toluene (reagent made by Junsei Chemical Co., Ltd.), 15 ml of ethanol (reagent made by Junsei Chemical Co., Ltd.), and 5 ml of desalted water. The pressure in the system was reduced, and the system was purged with nitrogen. This operation was repeated five times. Furthermore, nitrogen was passed into the reaction solution for 30 minutes to degas the solution. Then, 0.2 g of tetrakistriphenylphosphine palladium (0) was added thereto, and the resultant was heated and stirred in an oil bath at 80°C for 12 hours.
The generated precipitation was collected by suction filtration, and the solid was washed with MeOH. The collected solid was dissolved into toluene at a hot reflux temperature, and the solution was thermally filtered to remove inorganic salts. The filtrate was concentrated, and washed with MeOH to yield the intended product (yielded amount: 2.0 g, yield: 90%).

$^1H$ NMR(400MHz,$CDCl_3$) δ8.29 (d,2H,J=9.60),8.23 (d,2H,J=9.60),8.19 (s,4H),8.07 (s,2H),8.00 (s,2H),7.90 (dd, 4H,J=8.00),7.82 (dd,4H,J=8.00),7.53-7.39 (m,18H),7.30-7.23 (m,6H),2.48 (s,6H),2,47 (s,12H)
Mass(DEI)Obs.m/Z=1094 ($M^+$),Calc. for $C_{86}H_{62}$

(Measurement/calculation of carrier mobility, EL luminous efficiency, and PL luminous efficiency)

The carrier mobility, the EL luminous efficiency, and the PL luminous efficiency were each measured and calculated as follows:

[Carrier mobility $\mu$($cm^2$/Vs)]

A relational expression between the drain voltage ($V_d$) and the drain current of an organic semiconductor is represented by the following expression (1), and it increases linearly (linear area),

[Expression 1]

$$I_d = \frac{W}{L} \mu C_i \left[ (V_g - V_T) V_d - \frac{1}{2} V_d^2 \right] \qquad (1)$$

As $V_d$ increases, $I_d$ is saturated by the pinch-off of the channel, so that $I_d$ becomes a constant value (saturated area) and is represented by the following expression (2):.

[Expression 2]

$$I_d = \frac{W}{2L} \mu_{sat} C_i \ (V_g - V_T)^2 \qquad (2)$$

In the expressions (1) and (2), each of the symbols is as follows:

L: channel length [cm],
W: channel width [cm],
$C_i$: electrostatic capacity [F/cm$^2$] of the gate insulating film per unit area,
$\mu_{sat}$: mobility [cm$^2$/Vs] in the saturate area,
$I_d$: drain current [A],
$V_d$: drain voltage [V],
$V_g$: gate voltage [V], and
$V_T$: gate threshold voltage [V] (which represents the following point: in a graph obtained by plotting the 1/2 power of the drain current ($V_{dsat}^{1/2}$) versus the gate voltage ($V_g$) under a condition that the drain voltage ($V_d$) in the saturated area is constant, a point at which the asymptotic line therein intersects the transverse axis)

From the relationship between $I_d^{1/2}$ and $V_g$ in this saturated area, the mobility ($\mu$) in the organic semiconductor can be obtained.

In the present invention, under conditions that the pressure is set to the degree of vacuum of $5 \times 10^{-3}$ and the temperature is set to room temperature, using a Semiconductor Parameter Analyzer (HP4155C, Agilent), the drain voltage was operated from 10 V to -100 V at increments of -1 V, and the gate voltage was operated from 0 V to -100 V at increments of -20 V to calculate the mobility using the expression (2).

(EL luminous efficiency)

For the EL luminous efficiency next , the above-mentioned transistor elements were used, and operations were made to set the drain voltage from 10 V to -100 V at increments of -1 V, and set the gate voltage from 0 to -100 V at increments of -20 V, and light emitted from the elements was measured with a photon counter (4155C, Semiconductor Parameter Analyzer, manufactured by Newport Co.). The following expression (3) was used to convert the number of photons [CPS] obtained therein to the light fluxes [lw], and subsequently the following expression (4) was used to calculate the EL luminous efficiency $\eta_{ext}$.

[Expression 3]

$$X_{PC}[h\nu] = \frac{5.71\times10^{-11}\ (N_{PC}[CPS]-\text{base})\ \frac{4}{3}\pi r^3 / \frac{h}{3}\pi r^2}{1.04\times10^{6}} \qquad (3)$$

[Expression 4]

$$\eta_{ext} = (100\times1239.7/\lambda \times N_{PC} \times X_{PC}) / I_d \qquad (4)$$

In the expressions (3) and (4), each of the symbols is as follows:

$N_{PC}$: number of photons [CPS] measured with the photon counter (PC),
$X_{PC}$: numerical value obtained by converting the number of the photons to light fluxes [lw],
r: diameter [cm] of the cone or circle, and
h: distance [cm] between the photon counter and the sample.

(PL luminous efficiency)

The PL luminous efficiency was calculated by depositing each of the materials obtained in the invention on a quartz substrate to a thickness of 70 nm in the atmosphere of nitrogen to form a monolayer film, using an integrating sphere (IS-060, Labsphere Co.) to radiate a He-Cd laser (IK5651R-G, Kimmon Electric Co.) having a wavelength of 325 nm as an exciting ray, and then measuring a light emitting Multi-channel photodiode (PMA-11, Hamamatsu Photonics Co.) from the samples.

(Examples 1 and 2)

Next, organic EL elements illustrated in Fig. 9 were produced under the following conditions:

Hole injecting electrode 32: ITO (110 nm)
Hole transporting layer 33: α-NPD (50 nm)
Light emitting layer 34: CBP + Asymmetric pyrene based compound ((4-5) or (4-7) in Fig. 2(a)) (The content of the asymmetric pyrene based compound: 6% by weight of the entire light emitting layer) (40 nm)
Electron transporting layer 35: Bphen (20 nm)
Electron injecting layer (not shown): MgAg (100 nm)
Electron injecting electrode 36: Ag (10 nm)

About each of the resultant elements, the HOMO and LUMO levels, the PL peak, the EL peak (the wavelength of actually-seen emission), the PL luminous efficiency, the external luminous efficiency, and the luminous brightness were measured. The results are shown in Table 1.

(Comparative Example 1)

The same measurement as in Example 1 was made except that tetraphenylpyrene (reagent made by Aldrich Co.; abbreviated to "TPPy") was used as a pyrene compound. The results are shown in Table 1. In Table 1, the PL luminous efficiencies and the external luminous efficiencies are each a luminous efficiency as the light emitting layer 34 (the content of the asymmetric pyrene based compound: 6% by weight).

Table 1

| | | Example | | Comparative Example |
|---|---|---|---|---|
| | | 1 | 2 | 1 |
| Compound | | (4-5) | (4-7) | TPPy |
| HOMO/LUMO energy levels (eV) | | 5.6/2.6 | 5.3/2.4 | 5.6/2.7 |
| PL peak(nm) | | 448 | 468 | 454 |
| EL peak (nm) | | 433 | 446 | 433 |
| PL luminous efficiency (%) | | 86 | 83 | 88 |
| External luminous efficiency (%) | | 1.4 | 2.5 | 1.3 |
| Luminous brightness ($cd/m^2$) | $10mA/cm^2$(6.0V) | 76.63 | 253.40 | 70.81 |
| | $100mA/cm^2$(8.0V) | 627.30 | 2107.00 | 595.70 |

(Examples 3 to 4)

Next, light emitting transistor elements each illustrated in Figs. 5 and 6 were produced under the following conditions:

Source electrode 2 and Drain electrode 3: Electrodes (Au, thickness: 40 nm) each having a comb tooth shaped region made of twenty comb teeth were formed, and then the electrodes were arranged on an insulating film 5 to arrange the comb tooth shaped regions alternately, as illustrated in Fig. 8. At this time, a layer (1 nm) made of chromium was formed between the insulating film 5 and the two electrodes. About the channel regions (between the comb tooth shaped regions) at this time, the width was set to 25 μm and the length was set to 4 mm.
Insulating film 5: A silicon oxide film 300 nm in thickness was formed by vapor deposition.

Light emitting layer 1: A light emitting layer 1 was formed by evaporating the pyrene based compounds ((4-5) and (4-7) in Fig. 2(a)), obtained by the above-mentioned production processes, each independently to cover the surroundings of the insulating film, the source electrode 2 and the drain electrode 3.

About each of the resultant elements, the HOMO and LUMO levels, the fluorescence absorption wavelength, the PL luminous efficiency, the EL luminous efficiency, the luminous brightness and the carrier mobility were measured. The results are shown in Table 2.

(Comparative Example 2)

The same measurement as in Example 2 was made except that tetraphenylpyrene (reagent made by Aldrich Co.) was used as a pyrene compound. The results are shown in Table 2.

Table 2

| | Example | | Comparative Example |
|---|---|---|---|
| | 3 | 4 | 2 |
| Compound | (4-5) | (4-7) | TPPy |
| HOMO/LUMO energy levels (eV) | 5.6/2.6 | 5.3/2.4 | 5.6/2.7 |
| Fluorescence absorption wavelength (nm) | 448 | 468 | 505 |
| PL luminous efficiency (%) | 33 | 31 | 34 |
| EL luminous efficiency(%) | - | $7.4\times10^{-3}$ | $9.0\times10^{-2}$ |
| Luminous brightness (CPS) | - | $3.5\times10^{5}$ | $1.2\times10^{6}$ |
| Carrier mobility ($cm^2/V\cdot S$) | - | $4.9\times10^{-5}$ | $5.5\times10^{-5}$ |

## Claims

1. An asymmetric pyrene based compound represented by the following structural formula (1):

[Chemical formula 1]

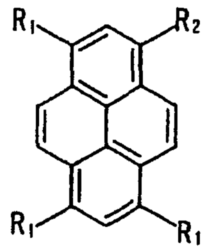

(1)

(wherein $R_1$ represents a group selected from a heteroaryl group which may have a substituent, an aryl group which may have a substituent, an alkyl group which may have a substituent and has a main chain having 1 to 20 carbon atoms, a cycloalkyl group which may have a substituent, an alkenyl group which may have a substituent, an alkynyl group which may have a substituent, a cyano group, a carbonyl group which may have a substituent, an alkoxy group which may have a substituent, an aryloxy group which may have a substituent, a silyl group which may have a substituent, an aryl boryl group which may have a substituent, an ester group which may have a substituent, and halogen atoms.
$R_2$ represents a group selected from a hydrogen atom, a heteroaryl group which may have a substituent, an aryl group which may have a substituent, an alkyl group which may have a substituent and has a main chain having 1 to 20 carbon atoms, a cycloalkyl group which may have a substituent, an alkenyl group which may have a substituent, an alkynyl group which may have a substituent, a cyano group, a carbonyl group which may have a substituent, an alkoxy group which may have a substituent, an aryloxy group which may have a substituent, a silyl group which may have a substituent, an aryl boryl group which may have a substituent, an ester group which may have a substituent, and halogen atoms, and is different from $R_1$.

2. The asymmetric pyrene based compound according to claim 1, wherein $R_1$ in the formula (1) is a group selected from phenyl, naphthyl, benzofuryl, phenylpyridyl, thienyl, benzothienyl, pyridyl, methyl, vinyl, and ethynyl groups, each of which may have a substituent, and $R_2$ is a group which is different from $R_1$ and selected from a hydrogen atom, and phenyl, naphthyl, benzofuryl, phenylpyridyl, thienyl, benzothienyl, pyridyl, methyl, vinyl, and ethynyl groups, each of which may have a substituent.

3. The asymmetric pyrene based compound according to claim 1 or 2, wherein $R_1$ in the formula (1) is a group selected from a 3-alkylphenyl, 4-alkylphenyl, 3-fluorophenyl, 4-fluorophenyl, 3-trifluoromethylphenyl, 4-trifluoromethylphenyl, 3,4-difluorophenyl, 3,5-difluorophenyl, 3,5-bis-(trifluoromethyl)phenyl, 3,4,5-trifluorophenyl, tolyl, fluorine-substituted phenyl, 2-naphthyl, benzofuryl, phenylpyridyl, thienyl, benzothienyl, pyridyl, bipyridyl, phenyl, biphenyl, methyl, phenyl-substituted vinyl, or phenyl-substituted ethynyl group, and
$R_2$ is a group which is different from $R_1$ and selected from a hydrogen atom, or a methyl, hexyl, phenyl, biphenyl, pyridyl, bipyridyl, naphthyl, biphenyl, phenylpyridyl, octyl, dodecyl, octadecyl, phenyl-substituted vinyl or phenyl-substituted ethynyl group.

4. An asymmetric pyrene based compound represented by the following formula (2):

[Chemical formula 2]

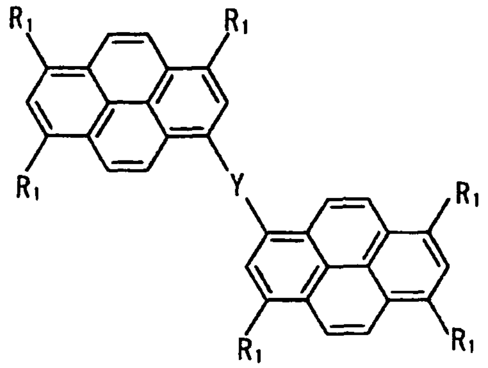

(2)

(wherein $R_1$ has the same meaning as described above, and Y represents a bivalent linking group.)

**5.** The asymmetric pyrene based compound according to claim 4, wherein Y is a group which may have a substituent and is selected from bivalent groups derived from aromatic heterocyclic rings, aromatic hydrocarbon rings, alkanes, alkenes, and alkynes.

**6.** The asymmetric pyrene based compound according to any one of claims 1 to 5 which is used in a light emitting transistor element or an organic electroluminescence element.

**7.** Alight emitting transistor element comprising:

a light emitting layer which is capable of transporting holes and electrons as carries, comprises the asymmetric pyrene based compound according to claim 6 as a main component, and emits light by recombination of the holes and the electrons,
a hole injecting electrode for injecting holes into this light emitting layer,
an electron injecting electrode for injecting electrons into the light emitting layer, and
a gate electrode for controlling the distribution of the carriers in the light emitting layer, the gate electrode being disposed opposite to the hole injecting electrode and the electron injecting electrode.

**8.** The light emitting transistor element according to claim 7 wherein the hole injecting electrode and the electron injecting electrode each have a comb tooth shaped region having a plurality of comb teeth, and the comb teeth which constitute the comb tooth shaped region of the hole injecting electrode and the comb teeth of the comb tooth shaped region of the electron injecting electrode are alternately arranged at predetermined intervals.

**9.** A display device wherein a plurality of the light emitting transistor elements according to claim 7 or 8 are arranged on a substrate.

**10.** An organic electroluminescence element comprising a light emitting layer which emits light by recombination of holes and electrons, a hole injecting electrode for injecting holes into this light emitting layer, an electron injecting electrode for injecting electrons into the light emitting layer, and a hole transporting layer and an electron transporting layer for transporting holes or electrons from the hole injecting electrode or the electron injecting electrode to the light emitting layer,
wherein the asymmetric pyrene based compound according to claim 6 is used as a component of the light emitting layer and as a component of at least one layer selected from the hole transporting layer and the electron transporting layer.

**11.** A display device wherein a plurality of the organic electroluminescence elements according to claim 10 are arranged on a substrate.

**12.** A process for producing an asymmetric pyrene based compound (1) by reacting 1-substituted pyrene (1-1) with a halide in accordance with the following reaction formula <1>:

[Chemical formula 3]

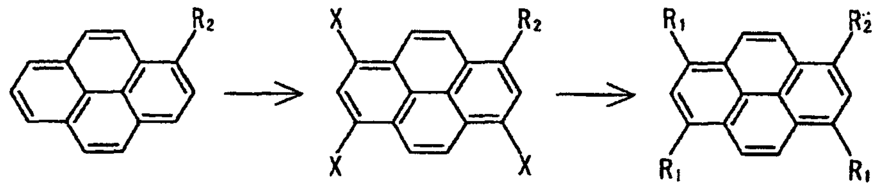

(1-1) (1-2) (1)

(1)

(wherein $R_1$ and $R_2$ are the same as in the case of the formula (1), and X represents a halogen atom selected from chlorine, bromine, and iodine atoms)
thereby synthesizing 1-substituted-3,6,8-trihalopyrene (1-2), and then reacting the resultant with an organometallic compound selected from boric acid compounds, tin compounds, organic zinc compounds, and organic magnesium compounds.

# Fig. 1(a)

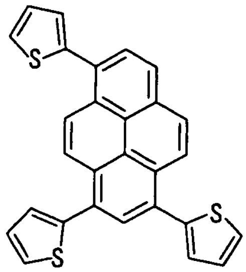

(3-1)

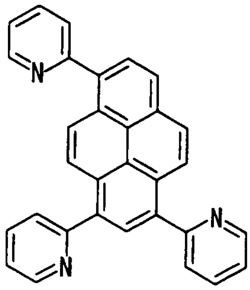

(3-2)

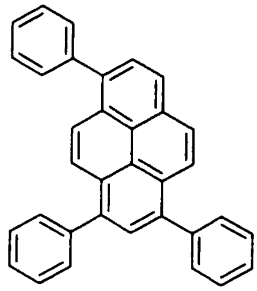

(3-3)

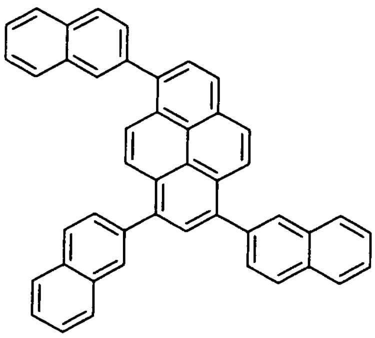

(3-4)

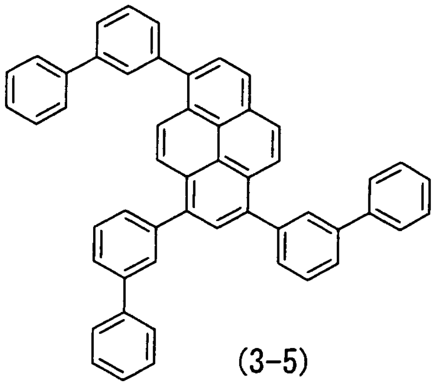

(3-5)

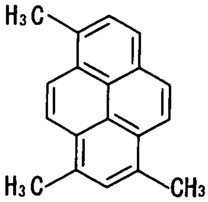

(3-6)

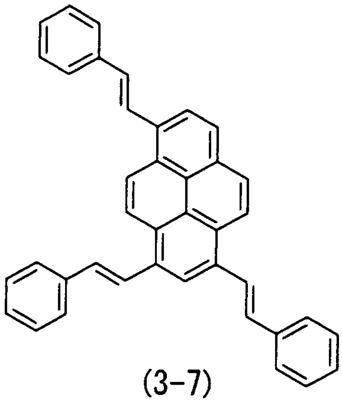

(3-7)

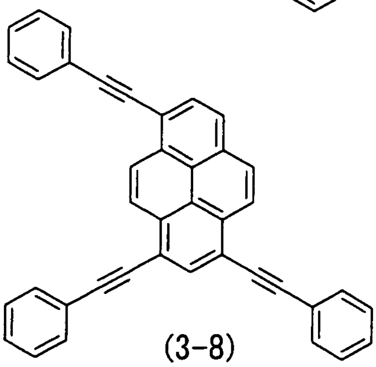

(3-8)

# Fig. 1(b)

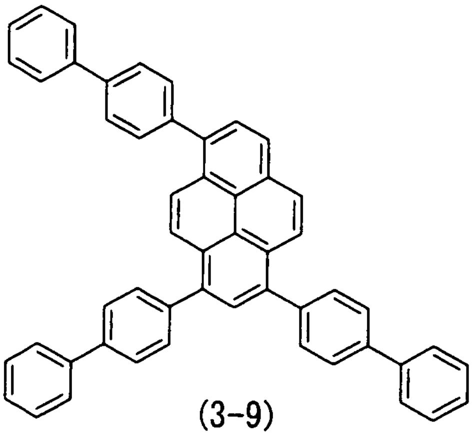

(3-9)

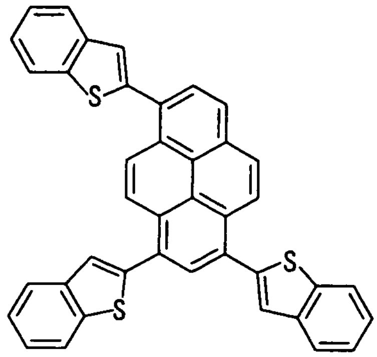

(3-10)

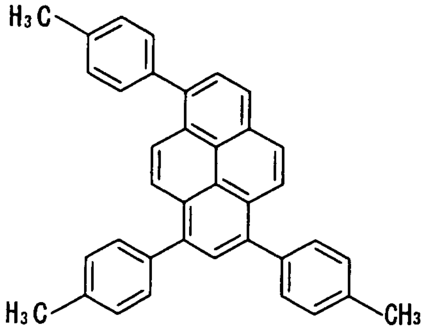

(3-11)

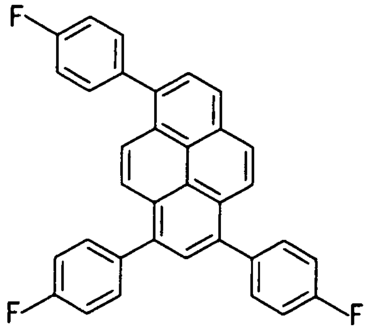

(3-12)

# Fig.2(a)

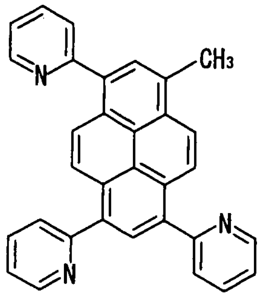

(4-1)

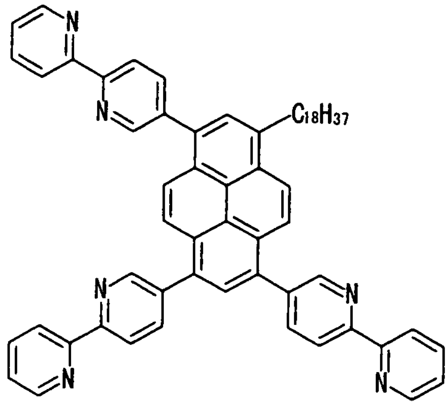

(4-2)

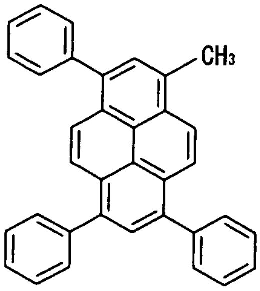

(4-3)

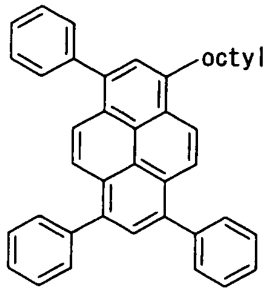

(4-4)

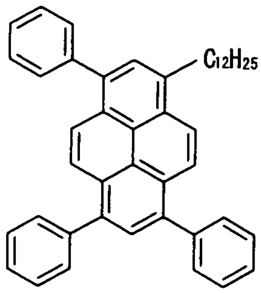

(4-5)

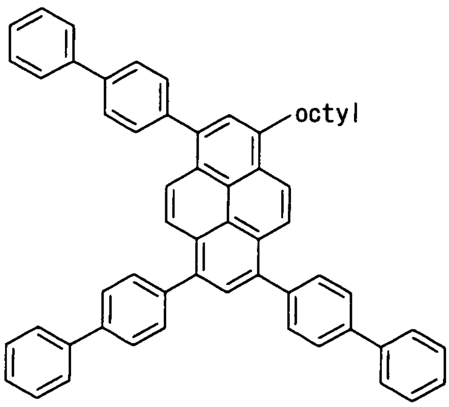

(4-6)

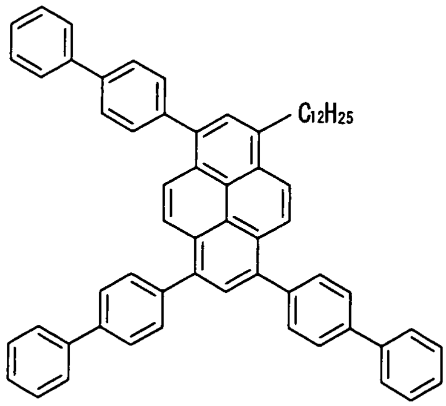

(4-7)

# Fig.2(b)

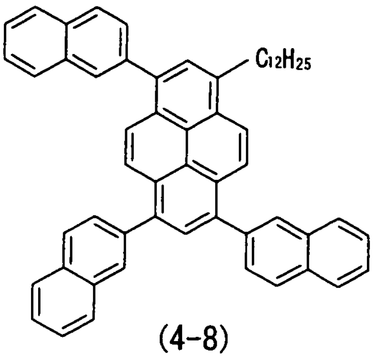

(4-8)

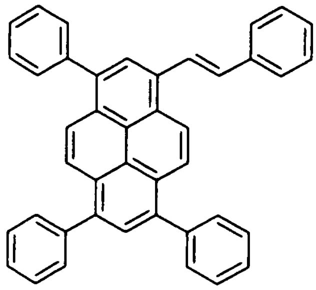

(4-9)

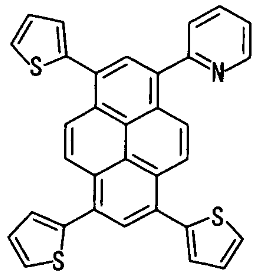

(4-10)

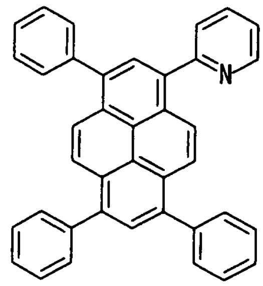

(4-11)

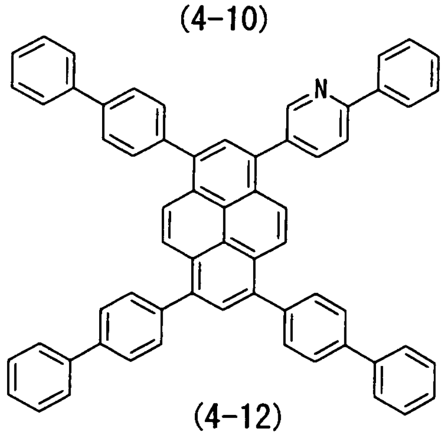

(4-12)

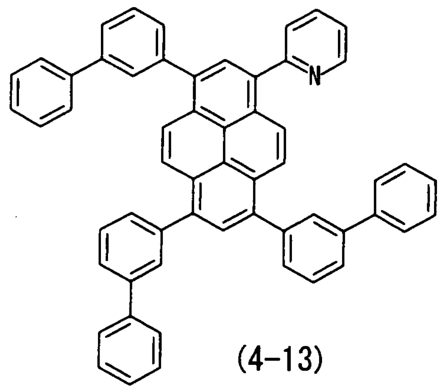

(4-13)

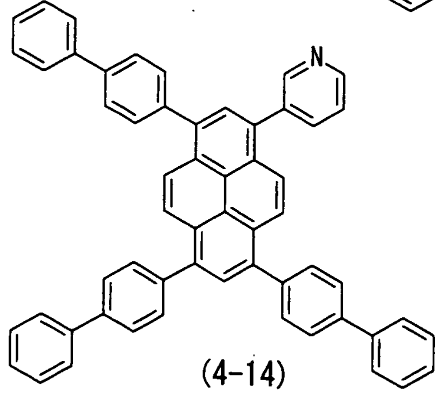

(4-14)

Fig.2(c)

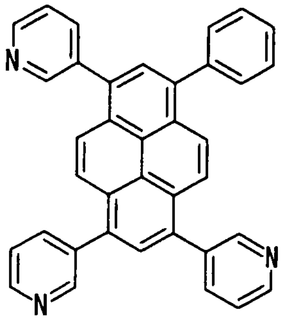

(4-15)

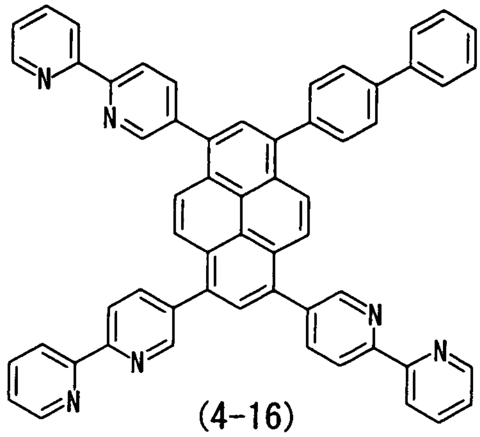

(4-16)

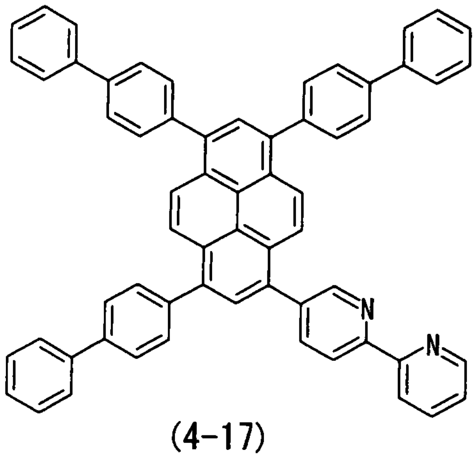

(4-17)

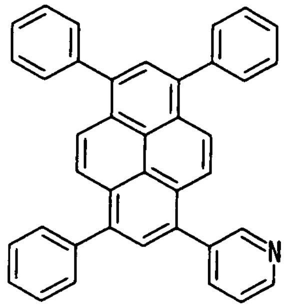

(4-18)

# Fig.3(a)

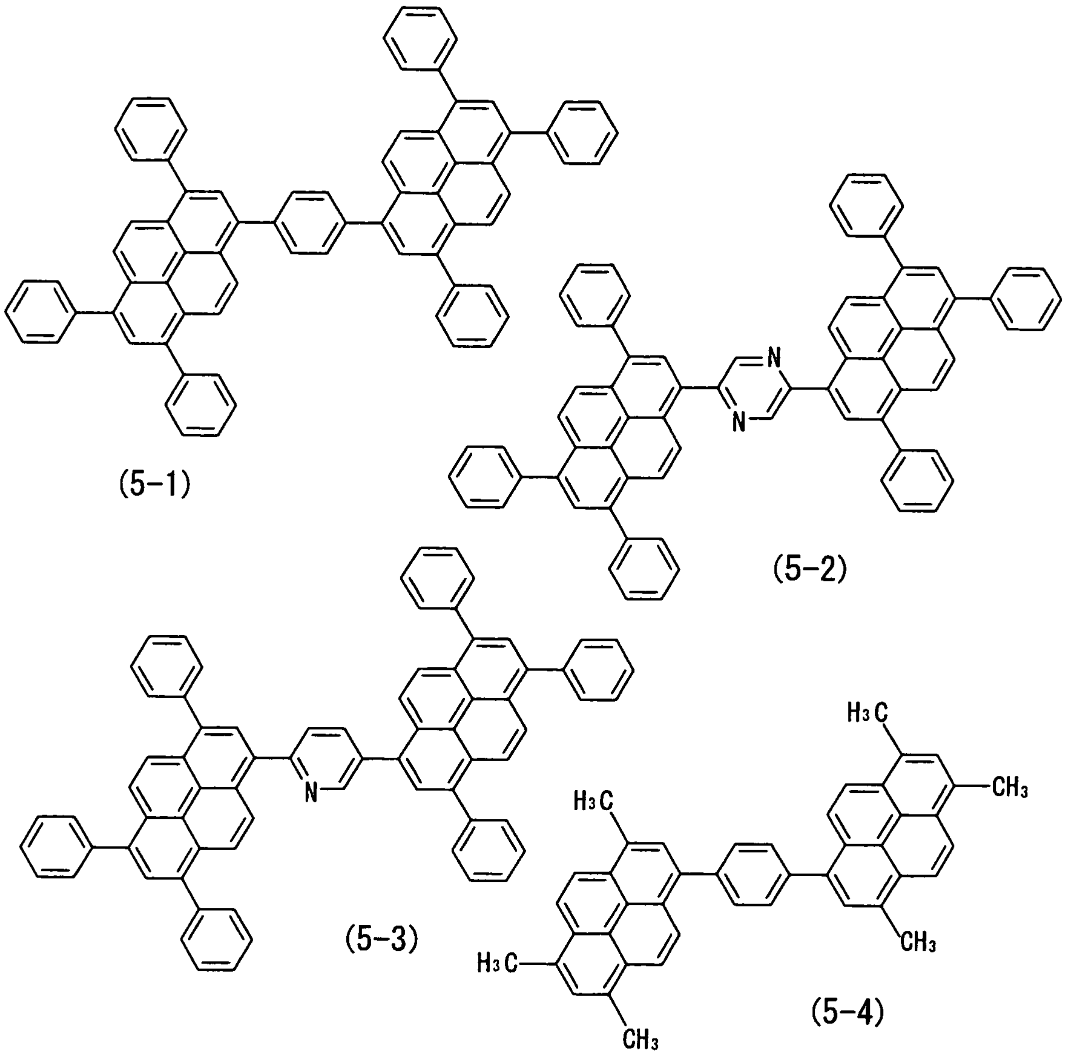

(5-1)

(5-2)

(5-3)

(5-4)

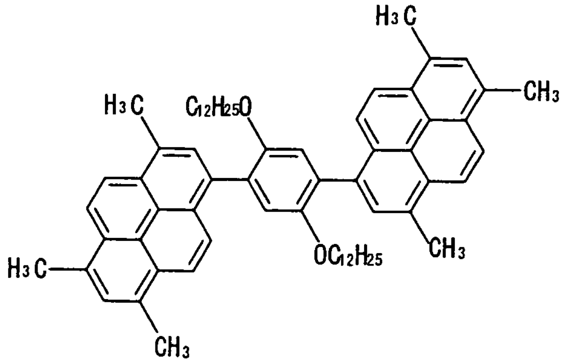

(5-5)

# Fig. 3(b)

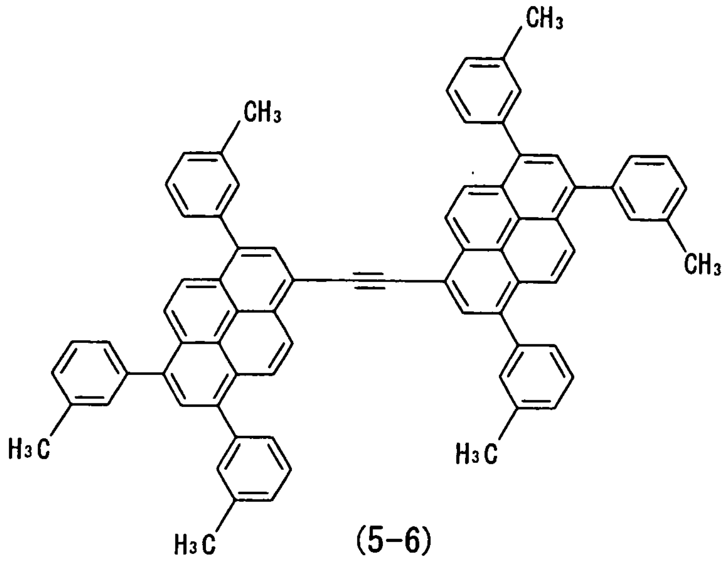

(5-6)

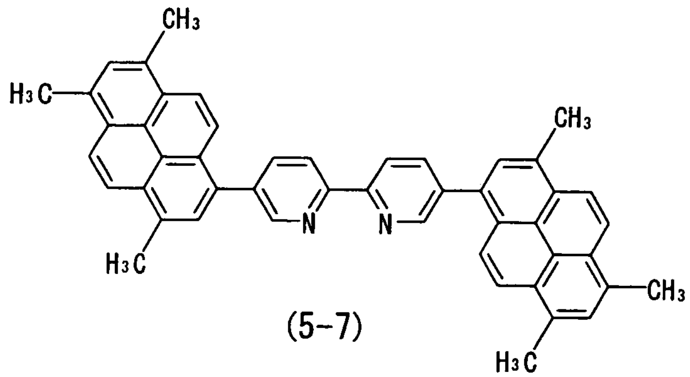

(5-7)

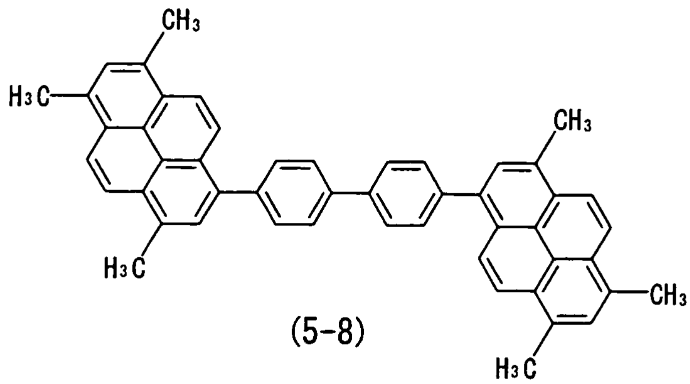

(5-8)

# Fig.3(c)

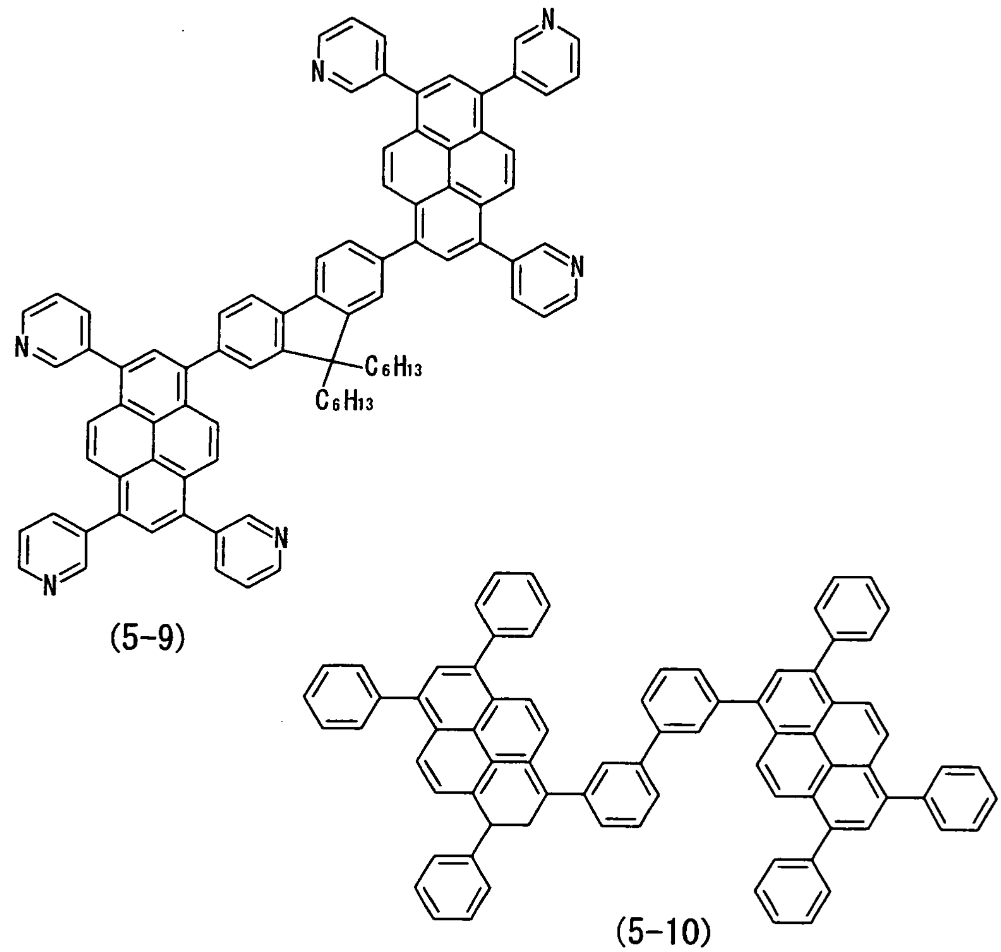

(5-9)

(5-10)

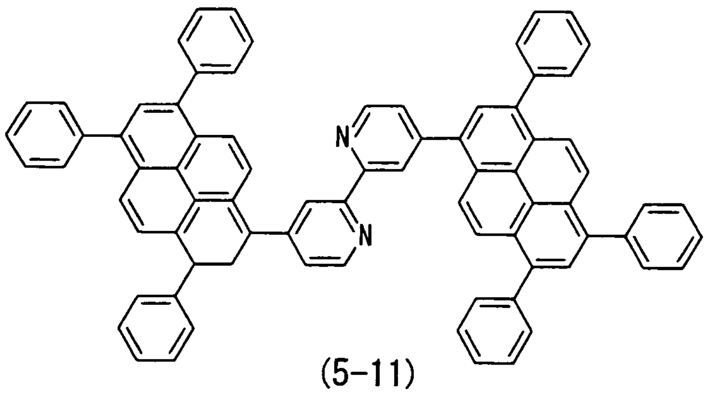

(5-11)

Fig.4(a)

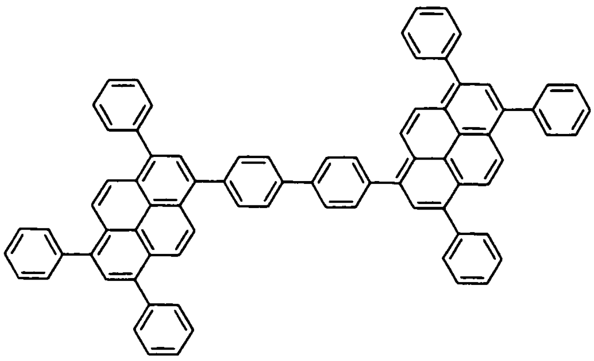

(6-1)

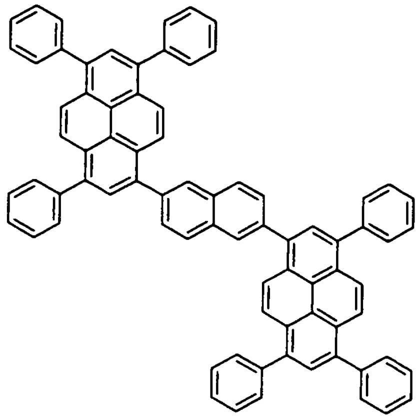

(6-2)

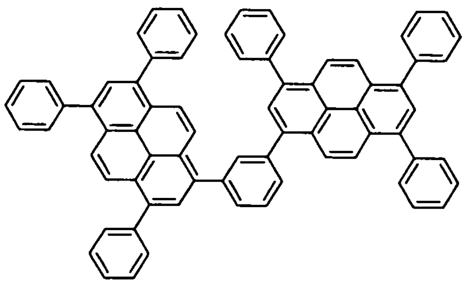

(6-3)

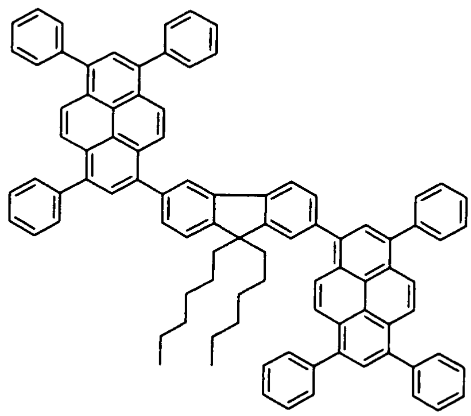

(6-4)

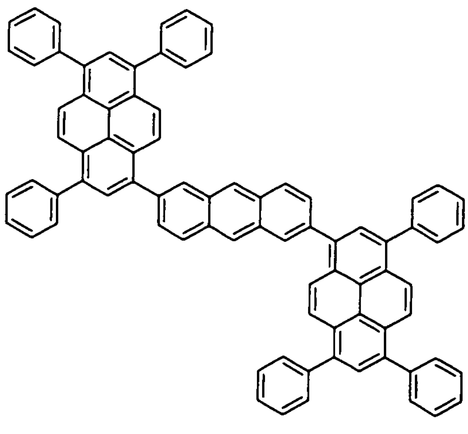

(6-5)

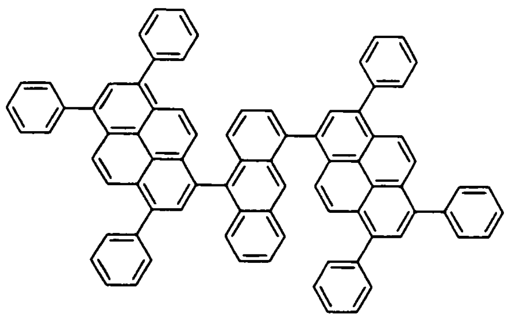

(6-6)

Fig.4(b)

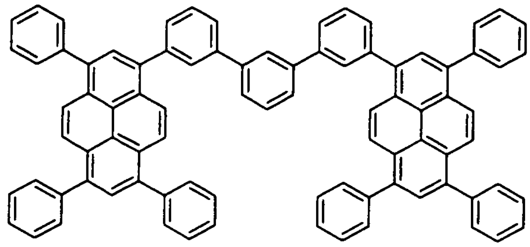

(6-7)

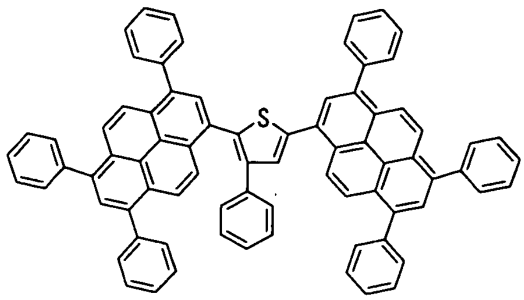

(6-8)

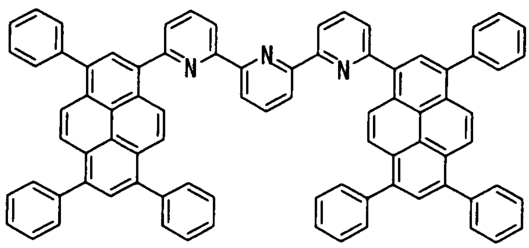

(6-9)

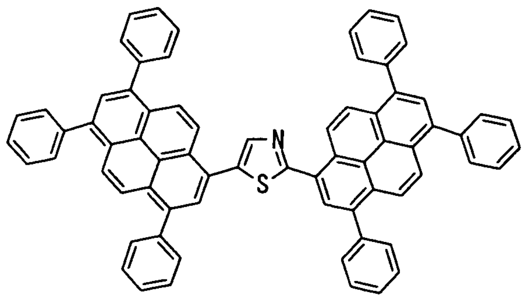

(6-10)

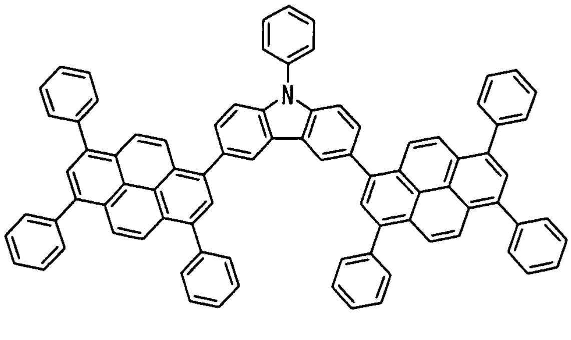

(6-11)

Fig.5

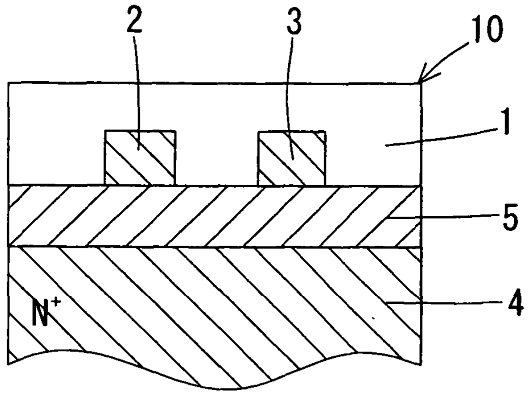


Fig.6

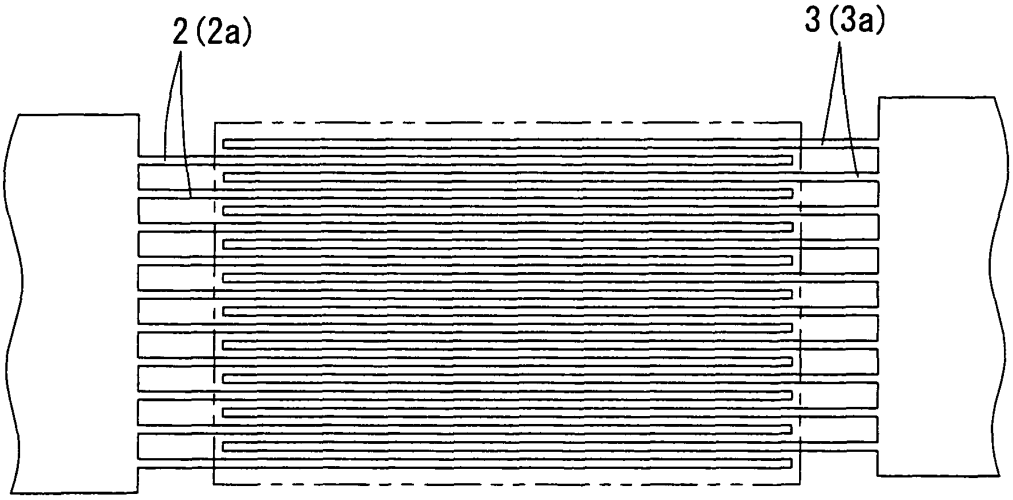

Fig.7

(a)

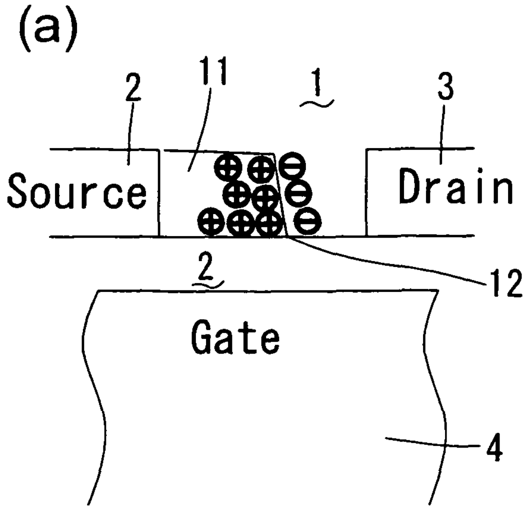


(b)

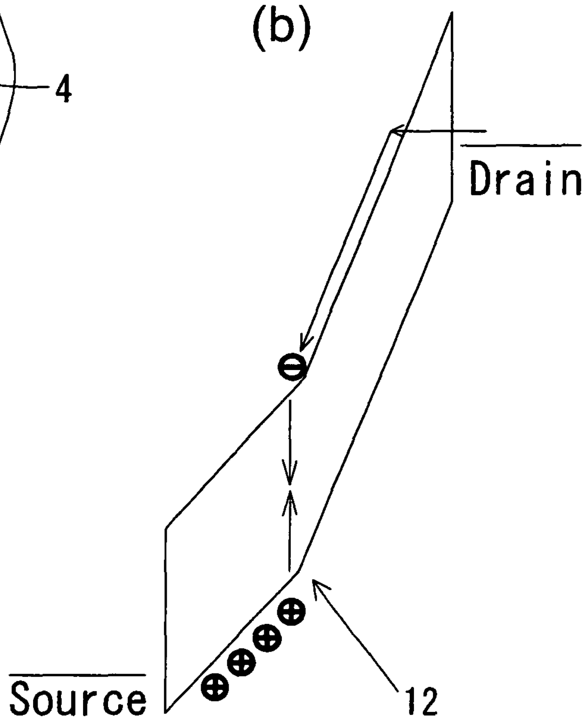


(c)

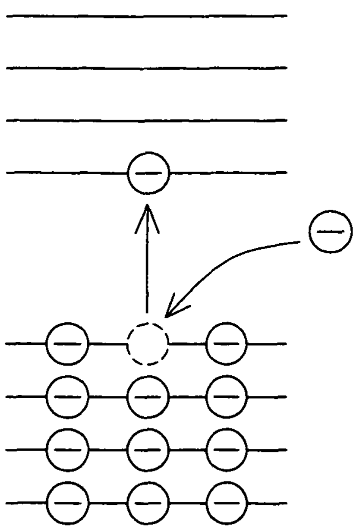

Fig.8

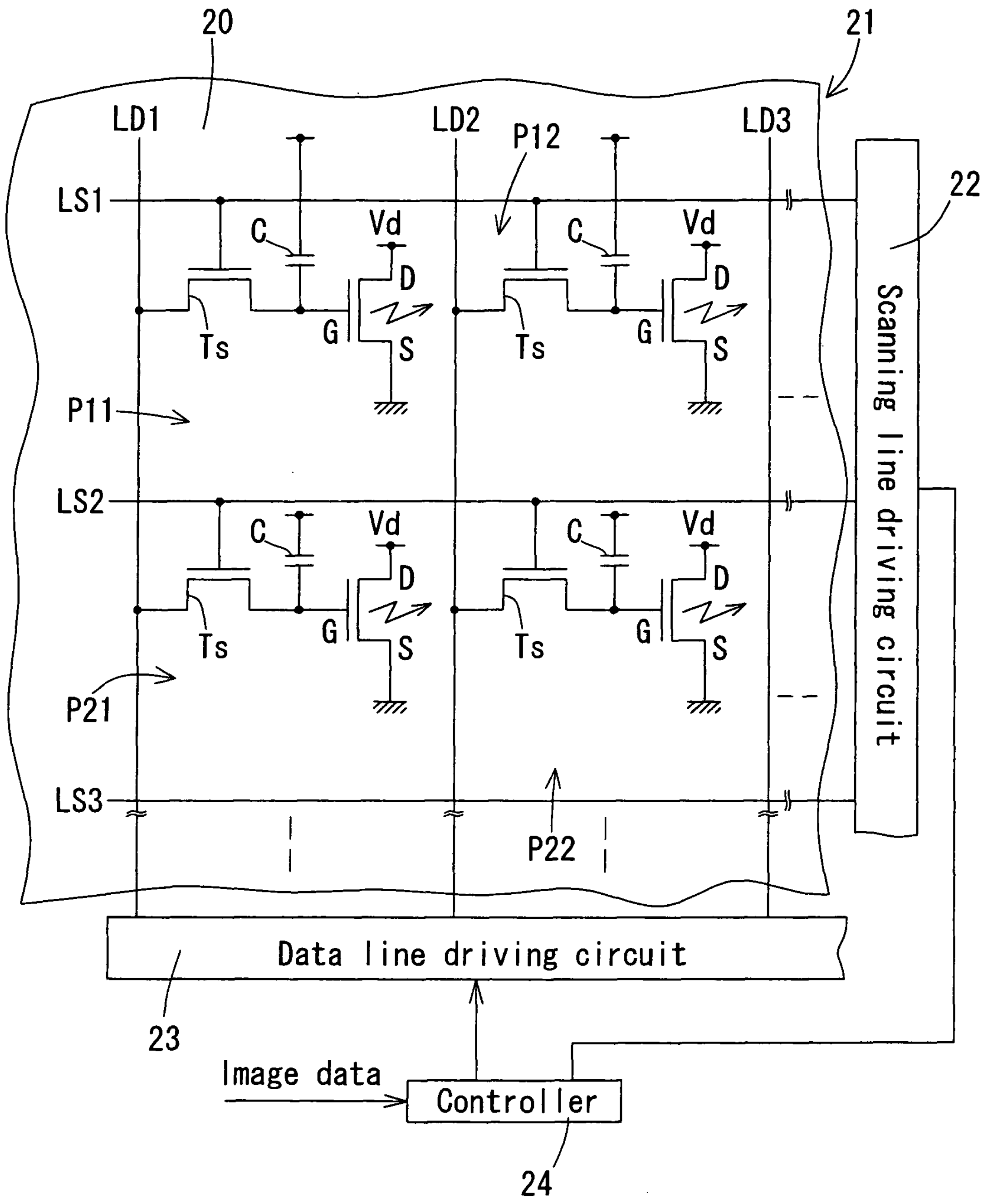
20
21
LD1
LD2
LD3
P12
LS1
LS2
LS3
22
C
Vd
D
G
S
Ts
P11
P21
P22
Scanning line driving circuit
Data line driving circuit
23
Image data
Controller
24

Fig.9

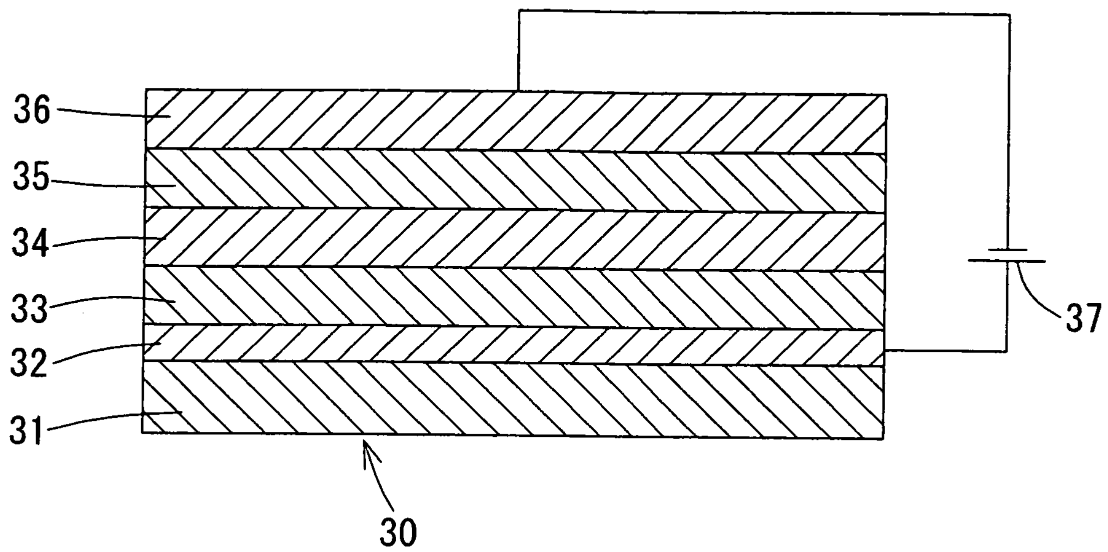

Fig.10

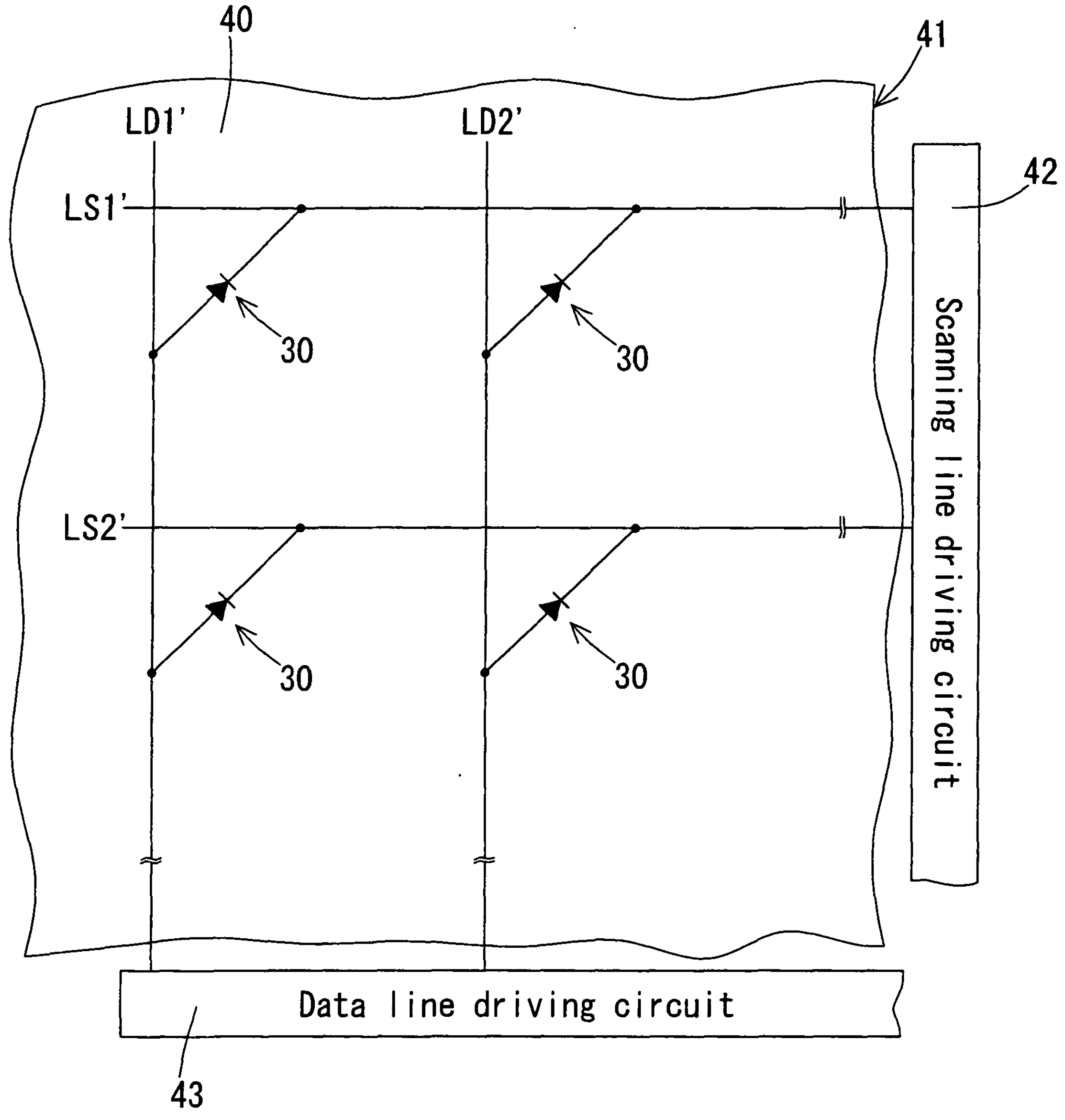
40
41
42
43
LD1'
LD2'
LS1'
LS2'
30
30
30
30
Scanning line driving circuit
Data line driving circuit

## INTERNATIONAL SEARCH REPORT

International application No.
PCT/JP2005/021647

A. CLASSIFICATION OF SUBJECT MATTER
***C07C15/38***(2006.01), ***C07C25/22***(2006.01), ***C07C1/32***(2006.01), ***C07C17/12***(2006.01), ***C07D213/53***(2006.01), ***C09K11/06***(2006.01), ***H01L51/50***(2006.01), ***C07D213/16***(2006.01), ***C07D213/22***(2006.01)

According to International Patent Classification (IPC) or to both national classification and IPC

B. FIELDS SEARCHED

Minimum documentation searched (classification system followed by classification symbols)
C07C1/32, C07C15/38, C07C17/12, C07C25/22, C07D213/16, C07D213/22, C07D213/53, C09K11/06, H01L51/50

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | Chemical Abstracts, Vol.73, abs.no.35116(1970) | 1-3 |
| X | Chemical Abstracts, Vol.120, abs.no.94411(1994) | 1 |
| X | Chemical Abstracts, Vol.77, abs.no.88143(1972) | 1 |
| X | Chemical Abstracts, Vol.136, abs.no.216806(2002) | 1 |
| X | Chemical Abstracts, Vol.69, abs.no.86693(1968) | 1 |
| X | Chemical Abstracts, Vol.32, abs.no.145i, 146a-i, 147a-i, 148a-i, 149a-i, 150a-i, 151a-i, 152a-e (1938) | 1 |
| X | Chemical Abstracts, Vol.131, abs.no.199379(1999) | 1 |
| X | Chemical Abstracts, Vol.105, abs.no.78625(1986) | 1 |

☒ Further documents are listed in the continuation of Box C.　☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>26 December, 2005 (26.12.05) | Date of mailing of the international search report<br>17 January, 2006 (17.01.06) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2005/021647

C (Continuation). DOCUMENTS CONSIDERED TO BE RELEVANT

| Category* | Citation of document, with indication, where appropriate, of the relevant passages | Relevant to claim No. |
|---|---|---|
| X | Chemical Abstracts, Vol.36, abs.no.87f-i, 88a-i (1942) | 1 |
| X | Chemical Abstracts, Vol.103, abs.no.87615(1985) | 1 |
| X | JP 2003-300912 A (Fuji Photo Film Co., Ltd.), 21 October, 2003 (21.10.03), Claim 1; Par. Nos. [0092], [0101], [0110], [0111] (Family: none) | 1,2,4-12 |
| X | JP 2001-192651 A (Fuji Photo Film Co., Ltd.), 17 July, 2001 (17.07.01), Claim 9; Par. Nos. [0060], [0069], [0079], [0080] & US 6635364 B1 & US 2004/48102 A1 | 1,2,4-6,10, 11 |
| X | JP 2001-118682 A (Fujitsu Ltd.), 27 April, 2001 (27.04.01), Claim 1; Par. No. [0013]; Fig. 3 (Family: none) | 6,10,11 |
| X | WO 2004/096945 A1 (Fujitsu Ltd.), 11 November, 2004 (11.11.04), Claim 1; page 11, line 1 to page 13, 6th line from the bottom (Family: none) | 6,10-12 |
| X | JP 2002-63988 A (Toray Industries, Inc.), 28 February, 2002 (28.02.02), Claims 1, 2; Par. Nos. [0013], [0025], [0026] (Family: none) | 6,10,11 |
| X | WO 2004/096743 A1 (Fujitsu Ltd.), 11 November, 2004 (11.11.04), Claims 1, 4; page 13, line 23 to page 17, line 22 & US 2005/156164 A1 | 6,10-12 |
| X | JP 9-241629 A (Idemitsu Kosan Co., Ltd.), 16 September, 1997 (16.09.97), Claims 1, 3; Par. No. [0014] (Family: none) | 6,10,11 |
| P,X | JP 2005-126431 A (Semiconductor Energy Laboratory Co., Ltd.), 19 May, 2005 (19.05.05), Claims 1, 2; Par. Nos. [0020], [0022] & US 2005/0079385 A1 | 6,10-12 |
| P,X | WO 2005/108348 A1 (Idemitsu Kosan Co., Ltd.), 17 November, 2005 (17.11.05), Page 44, lines 19 to 20 (Family: none) | 1 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5315078 A
- JP 2001118682 A